# EUROPÄISCHE PATENTANMELDUNG

(11) **EP 1 985 706 A2**
(43) Veröffentlichungstag der Anmeldung: **29.10.2008**
(21) Anmeldenummer: 08103225.2
(22) Anmeldetag: 20.12.2000
(51) Int. Cl.: C12N 15/62, C12N 15/11, C07K 14/705, A61K 38/17

(54) **BI- ODER OLIGOMER EINES DI-, TRI-, QUATTRO- ODER PENTAMERS VON REKOMBINANTEN FUSIONSPROTEINEN**

(30) Priorität: 30.12.1999 DE 19963859
(62) Teilanmeldung aus: 00987425.6
(71) Anmelder: Topotarget Switzerland SA, 1004 Lausanne (CH)
(72) Erfinder: Tschopp, Jurg, 1066 Epalinges (CH); Schneider, Pascal, 1066 Epalinges (CH); Holler, Nils, 3063 Ittigen (CH)
(74) Vertreter: Tetaz, Franck Claude Edouard

(57) **Zusammenfassung**

Die Erfindung betrifft Oligomere eines Di-, Tri-, Quattro- oder Pentamers von rekombinanten Fusionsproteinen, die dadurch gekennzeichnet sind, daß die rekombinanten Fusionsproteine mindestens eine Komponente A und mindestens eine Komponente B aufweisen, wobei Komponente A ein Protein oder einen Proteinabschnitt mit biologischer Funktion, insbesondere mit Ligandenfunktion für Antikörper, für lösliche oder membranständige Signalmoleküle oder für Rezeptoren oder einen Antikörper oder Abschnitt eines Antikörpers, umfaßt und Komponente B ein Protein oder einen Proteinabschnitt unfaßt, das/der das Di-, Tri-, Quattro- oder Pentamer des rekombinanten Fusionsproteins ohne Wirkung von Drittmolekülen bi- oder oilgomerisiert. Die Erfindung betrifft weiterhin die Verwedung derartiger Bi- oder Oligomere zur Herstellung eines Arzneimittels sowie die Fusionsproteine, die sich zu Bi- oder Oligomeren Zusammenlagern, deren DNA- Sequenz und Expressionsverktoren bzw. Wirtszelle mit dieser DNA-Sequenz.

## Beschreibung

Die vorliegende Erfindung betrifft Bi- oder Oligomere von Bi-, Tri-, Quattro- oder Pentameren von rekombinanten Fusionsproteinen, wobei die rekombinanten Fusionsproteine mindestens eine Komponente A und mindestens eine Komponente B aufweisen. Weiterhin betrifft die vorliegende Erfindung die Verwendung von derartigen Bi- oder Oligomeren zur Herstellung eines Arzneimittels bzw. deren Verwendung zur In-vitro-Diagnose. Schließlich betrifft die vorliegende Erfindung auch Fusionsproteine, die eine Komponente A und eine Komponente B aufweisen, wobei die Komponente B einen multimerisierenden und oligomerisierenden Abschnitt oder ein funktionelles Derivat eines solchen Abschnitts eines Proteins, ausgewählt aus der Gruppe, bestehend aus der Familie der C1q-Proteine oder der Collectine, enthält. Auch DNA-Sequenzen, die für ein derartiges Fusionsprotein kodieren, sowie Expressionsvektoren und Wirtszellen, die die DNA-Sequenz bzw. den Expressionsvektor enthalten, sind Gegenstand der vorliegenden Erfindung.

Proteine, die physiologisch als Di-, Tri-, Quattro- oder Pentamere auftreten, sind in der Natur in großer Zahl vorhanden. Aufgrund von Wechselwirkungen an den Oberflächen dieser in Lösung di- oder multimerisierenden Proteine kann es zu spontanen oder aber auch zu bspw. kinetisch verzögerten, weil konzentrations- oder milieuabhängigen Zusammenlagerungen von Proteinen kommen. Hierfür sind hydrophobe Wechselwirkungen, Wasserstoffbrückenbindungen und/oder Coulomb-Kräfte verantwortlich.

Daneben aber sind bei gewissen Proteinen Strukturmotive anzutreffen, die zur Bildung von spezifischen strukturbedingten intermolekularen Supersekundärstrukturen und damit zu Proteindi- oder -multimeren führen. Die Formation von Supersekundärstrukturen beruht auf charakteristischen Aminosäuresequenzen der diese Di- oder Multimere bildenden Proteine. Als Supersekundärstrukturen wären beispielsweise sog. "Coiled-Coil-Helices" zu nennen, die eine Di- oder Multimerisierung von Proteinen durch Interaktionen von charakteristischen α-Helices, die bei jedem der die Coiled-Coil-Form ausbildenden Proteine auftreten, bewirken. Die Coiled-Coil-Helix als intermolekulare "Di- oder Multimerisierungsdomäne" von Proteinen stellt sich strukturell als zwei- oder mehrsträngige, umeinander gewundene Superhelix dar. Derartige Coiled-Coil-Motive treten insbesondere bei extrazellulären Proteindi- oder -multimeren auf, ganz besonders aber bei Proteinen bzw. Proteinkomplexen des Bindegewebes.

So etwa beschreiben Beck et al. (J. Mol. Biol. (1996) 256, 909-923) ein Bindegewebsprotein, das sog. Cartilage Matrix Protein (CMP), dessen Aggregation zu einem Homotrimer auf Tripelhelix, die das Resultat der Zusammenlagerung von drei komplementären Helices (jeweils als Bestandteil eines Polypeptids) darstellt, mit Coiled-Coil-Muster beruht. Charakteristisch für die Aminosäuresequenz einer solchen eine Tripelhelix bildenden Helix ist dabei das Heptadmuster (abcdefg)ₙ. Deren Aminosäuren in den Positionen a und d tragen gewöhnlich apolare Seitenketten und erlauben dadurch die Ausbildung der oben beschriebenen superhelikalen Struktur, hier als Tripelhelix aus drei Helices.

Darüber hinaus findet sich in der Literatur beschrieben, daß bei einem anderen extrazellulären Matrixprotein (Cartilage Oligomeric Matrix Protein (COMP)) sogar fünf Helices in Form einer fünfsträngigen Coiled-Coil-Helix miteinander interagieren und damit Pentamere ausbilden können. (Kajava, PROTEINS: Structure, Function and Genetics, 24:218-226 (1996); Malashkevich et al., Science, Vol. 274, 761-765, 1996).

Neben den Matrixproteinen COMP und CMP, die nicht zu den Proteinen aus der Kollagenfamilie gehören, werden weiterhin auch bei Proteinen aus der Kollagenfamilie spezifische strukturbedingte Multimerisierungsphänomene durch die Ausbildung von Supersekundärstrukturen angetroffen. Dabei ist die Struktur von Kollagenfasern durch das Tropokollagen gekennzeichnet, das aus drei helikalen verdrillten Polypeptiden besteht. Auch die Protofibrille eines Haares ist aus einer Tripelhelix aus α-Keratin mit dem Motiv "Coiled-Coil", allerdings linksgängig, aufgebaut.

Um die Avidität von Liganden zu erhöhen, haben Terskikh et al. (PNAS, Vol. 94, 1663-1668, 1997) vorgeschlagen, Fusionsproteine einzusetzen, die ein kurzes Peptid mit Ligandenfunktion und die "Coiled-Coil-Domäne" des Matrixproteins COMP aufweisen. Für diese Pentamere konnte eine erhöhte Avidität nachgewiesen werden, wobei Aggregate höherer Ordnung auf diesem Weg nicht erhalten werden konnten.

Darüber hinaus sind aufgrund ihrer Sequenzhomologien in ihren jeweiligen multimerisierenden Sequenzabschnitten die Proteine C1q, Kollagen α1 (X), α2 (VII), das Überwinterungsprotein, ACRP30, das innere Ohrstrukturprotein, das Cerebellin und das Multimerin als Proteinfamilie unter der Bezeichnung Clq-Familie zusammengefaßt worden (Kischore und Reid, Immunopharmacol., 42 (1999) 15-21), die gleichfalls strukturbedingt als Di- oder Multimere vorliegen. Unter den in dieser Familie auftretenden Proteinen mit Multimerisierungseigenschaften ist bspw. die Struktur des aus dem Komplementsystem bekannten Proteins C1q durch Monomere charakterisiert, die jeweils eine globuläre sog. "Kopf"-Domänen und einen "kollagenähnlichen" helikalen Sequenzabschnitt aufweisen. Über diesen helikalen Sequenzabschnitt, der eine Coiled-Coil-Tripelhelix bildet, trimerisieren die Monomere. Sechs dieser Clq-Trimere formen wiederum ein Oligomer, wobei wiederum die Oligomerisierung der Proteintrimere auf Interaktionen zwischen den einzelnen Coiled-Coil-Tripelhelices beruht. Im Ergebnis führt diese strukturelle Anordnung beim Protein bzw. multi- (oligo-) merisierten Proteinkomplex C1q zu einem als "Bouquet" bezeichneten Aufbau, wobei sichergestellt wird, daß 18 globuläre, C-terminal angeordnete "Kopf"-Domänen zu einem Hexamer von Trimeren verbunden werden.

Eine ähnliche Struktur wie beim Protein C1q ist auch beim Protein ACRP30, gleichfalls ein Protein aus der C1q-Familie, anzutreffen (Hu et al., J. Biol. Chem., Vol. 271, Nr. 18, 10697-10703, 1996). Bei diesem von Adipozyten sekretierten Serumprotein handelt es sich mit überwiegender Wahrscheinlichkeit um Quattromere von Trimeren, wobei, wie auch beim Clq-Protein, globuläre C-terminale Domänen über kollagenähnliche Tripelhelices verbunden werden. Mutmaßlich vier dieser Tripelhelices formen schließlich wiederum ein Oligomer durch entsprechende Interaktionen. In der Veröffentlichung von Shapiro und Scherer (Current Biology 1998, 8:335-338) findet sich die mit Hilfe der Röntgenstrukturanalyse ermittelte Struktur eines Homotrimers von ACRP30 dargestellt.

Weiterhin sind aus der Literatur Proteine aus der Klasse der Collectine bekannt, die durch eine kollagenartige Domäne, eine Halsregion und darüber hinaus durch eine globuläre carboxyterminale Lectinbindungsdomäne charakterisiert sind. Auch die Collectine treten physiologisch als Oligomere von Trimeren auf. So trimerisieren beispielsweise die Proteine Lung Surfactant Protein A (SP-A) und das Mannose-Bindungsprotein (MBP), jeweils aus der Familie der Collectine, durch die Interaktionen ihrer "kollagenähnlichen" Domäne und liegen schließlich als Hexamere von Trimeren vor (Epstein et al., Current Opinion in Immunology, Vol. 8, Nr. 1, 1996, 29-35). Demgemäß bilden also auch die unter der Bezeichnung Collectine bekannten Proteine Oligomere (bspw. Hexamere) von Multimeren (bspw. Trimere) aus.

Aus der Literatur ist zudem zu entnehmen, daß zahlreiche physiologisch als Signalmoleküle wirkende Proteine ein biologisches Signal nur in bestimmten Zuständen transduzieren können. So ist beispielsweise membranständig angeordnetes FasL biologisch, d.h. apoptotisch, wirksam, während nach Abspaltung des extrazellulären Proteinabschnitts von dem membranständigen Abschnitt (sog. sFasL) diese nichtmembrangebundene sFasL-Fraktion physiologisch keine apoptotische Wirkung auf Zielzellen mehr hervorrufen kann. In der Veröffentlichung von Schneider et al. (J. Exp. Med., Vol. 187, Nr. 8, 1998, 1205-1213) wurde beschrieben, daß die biologische Wirkung von sFasL-Trimeren, die - wie zuvor erläutert - nach Abspaltung vom membranständigen Proteinabschnitt erhalten werden, gleichwohl durch den Einsatz von vernetzenden Antikörpern in Hinblick auf deren physiologische Funktion reaktiviert werden kann. Hierzu wurde ein Fusionsprotein, bestehend aus der Trimerisierungsdomäne von FasL, einer kurzen Linkersequenz und einer Flag-Markierung (mit der Flag-Aminosäuresequenz (Ein-Buchstaben-Code) DYKDDDDK) konstruiert, exprimiert, und derartige nichtstrukturbedingt (also nicht über spezifische Sekundärstruktur-Interaktionen mit dem Ergebnis der Bildung einer Supersekundärstruktur) trimerisierte Fusionsproteine durch gegen die Flag-Markierung gerichtete Antikörper vernetzt.

Derartig durch Antikörperbindung vernetzte sFasL-Moleküle zeigen eine signifikante Erhöhung der spezifischen apoptotischen Aktivität gegenüber nicht-vernetzten sFasL-Trimeren. Diese von Schneider et al. vorgeschlagene Vorgehensweise weist jedoch den Nachteil auf, daß neben den rekombinanten, nicht-membrangebundenen FasL-Proteinen mit der Trimerisierungsdomäne auch spezifische Antikörper eingesetzt werden müssen, also eine Erhöhung der biologischen Aktivität nur durch die Bereitstellung einer weiteren Molekülfraktion zu errreichen ist. Darüber hinaus ist es durch die von Schneider et al. vorgeschlagenen Lehre nicht möglich, einen exakt vorgegebenen oder bestimmbaren Oligomerisierungsgrad der Multimere sicherzustellen. Die Antikörper können nämlich bewirken, daß sich die FasL-Trimere zu Dimeren verbinden oder aber auch ein breites Spektrum von oligomerisierten Komplexen bis hin zu riesigen sFasL-/Antikörper-Aggregaten auftritt. Da bspw. für die medizinischen Anwendung ein genau definiertes Produkt mit maximaler Wirksamkeit gefordert wird, ist im Ergebnis daher der von Schneider et al. vorgeschlagene Weg zur Reaktivierung bzw. der Erhöhung der sFasL-Aktivität nicht praktikabel.

Eine zentrale Aufgabe der vorliegenden Erfindung ist es nunmehr, Verbindungen zur Verfügung zu stellen, die die Nachteile des Stands der Technik vermeiden, insbesondere auch eine gesteigerte biologische Aktivität aufweisen oder aber eine Reaktivierung der biologischen Aktivität bewirken.

Die vorliegende Aufgabe wird durch den Gegenstand des Anspruchs 1, nämlich Bi- oder Oligomere eines Di-, Tri-, Quattro- oder Pentamers von rekombinanten Fusionsproteinen, dadurch gelöst, daß die rekombinanten Fusionsproteine mindestens eine Komponente A und mindestens eine Komponente B aufweisen, wobei die Komponente A ein Protein oder einen Proteinabschnitt mit biologischer Funktion, insbesondere mit Bindungsfunktion, umfaßt und die Komponente B ein Protein oder einen Proteinabschnitt umfaßt, das/der das Di-, Tri-, Quattro- oder Pentamer eines rekombinanten Fusionsproteins mit biologischer Funktion ohne Wirkung von Drittmolekülen bi- oder oligomerisiert oder aber Fusionsproteine zu Di- oder Multimeren aggregiert und diese Di- oder Multimere gleichzeitig ohne Wirkung von Drittmolekülen zu einem Bi- oder Oligomer verbindet.

In der Darstellung der vorliegenden Erfindung werden die Begriffe Di-, Tri-, Quattro- oder Pentamer unter der Bezeichnung Multimer zusammengefaßt, und es werden hierunter-Proteinkomplexe aus zwei, drei, vier oder fünf zusammengelagerten Polypeptiden (Proteinen) verstanden. Dagegen werden die Aggregate der nächsthöheren Ordnung, also die Aggregationen von zwei oder mehr Di-, Tri-, Quattro- oder Pentameren im obigen Sinn als Bi- oder Oligomere bezeichnet. Unter einem Protein oder Proteinabschnitt mit biologischer Funktion (Komponente A im Fusionsprotein) sind insbesondere Proteine, die eine Ligandenfunktion, ganz besonders für Antikörper oder Rezeptoren, haben (also als Bindungspartner mit einem oder mehr Molekül(en) in Wechselwirkung treten können), modifizierte Aminosäuresequenzen, z.B. Aminosäuresequenzen mit kovalent oder nicht-kovalent angekoppelten Wirkstoffen (ggf. organisch-chemischer Natur), Antikörper oder Abschnitte von Antikörpern mit Paratopen oder auch Hormone, bspw. Peptidhormone, zu verstehen. Ganz besonders umfaßt die vorliegende Erfindung Aminosäuresequenzen von Signalproteinen, die biologisch bereits als Monomere aktiv sind und entsprechend als Bestandteile in einem erfindungsgemäßen Komplex in ihrer Wirkung gesteigert werden oder aber durch ihre erfindungsgemäß eingeleitete Multi- und Oligomerisierung oder erfindungsgemäß ausschließlich eingeleitete Oligomerisierung (sofern bereits Komponente A des Fusionsproteins als Trimer vorliegt) erst aktiv werden, als Komponente A im Fusionsprotein. Bei physiologisch membranständigen Signalproteinen, bspw. bei TNF-Cytokinen, werden Spaltprodukte, die die extramembranösen, insbesondere die extrazellulären Proteinabschnitte, umfassen, bevorzugt. Aber auch Aminosäuresequenzen, die als Antigene wirken können, können als Komponente A im rekombinanten Fusionsprotein eingesetzt werden. Schließlich können als Komponente A auch Rezeptoren, z.B. Rezeptoren aus der TNF-Rezeptorfamilie, etwa zugehörig zur Familie der TypI-Membranproteine (bspw. FasR), oder Abschnitte bzw. Derivate solcher Rezeptoren zum Einsatz kommen, die gleichfalls Bindungsfunktion haben (damit also als Bindungspartner mit einem anderen Molekül in Wechselwirkung treten) und i.S. der vorliegenden Erfindung daher auch unter den Begriff "Ligand" fallen. Derartige bindungsfähige Fragmente von biologischen Rezeptoren eigenen sich insbesondere zur Verwendung als Arzneimittel, wenn der komplementäre biologische Ligand beim Patienten in unphysiologisch hohe Konzentrationen vorliegt.

In einer bevorzugten Ausführungsform können die Komponenten A die in erfindungsgemäßen Oligomeren vorliegenden Multimere, also Di-, Tri-, Quattro- oder Pentamere, identische Komponenten A (Oligomere von Homodi- oder -multimeren) oder unterschiedliche Komponenten A (Oligomere von Heterodi-oder -multimeren) aufweisen. Auf diese Weise können Proteine mit verschiedenen Komponenten A, ggf. mit unterschiedlicher biologischer Funktion, in Di- oder Multimeren von erfindungsgemäßen Oligomeren verbunden sein. Die einzelnen in dem Bi- oder Oligomer aggregierten Heterodi- oder Heteromultimere können dann ebenfalls gleich oder verschieden sein, d.h. ein erfindungsgemäßes Bi- oder Oligomer kann sich ggf. auch aus verschiedenen Heterodi- oder -oligomeren zusammensetzen.

Allerdings ist es auch möglich, daß die Fusionsproteine im jeweiligen Di- oder Multimer als Untereinheit des Bi- oder Oligomers identisch sind, dagegen die einzelnen als Di-oder Multimer ausgestalteten Untereinheiten im Bi- oder Oligomer verschieden sind (Heterobi- oder Heterooligomer von Homodi-, -tri-, -quattro- oder -pentameren). Derart können beispielsweise in einem erfindungsgemäßen Hexamer von Trimeren bis zu sechs in bezug auf die Komponente A verschiedene Homotrimere assoziiert werden. Auf diese Weise werden durch die Wahl, die Anordnung, die spezifische Kombination und/oder durch die Anzahl der Komponenten A im Bi-oder Oligomer typischerweise fein modulierte biologische Aktivitäten bewirkt werden können. Es ist bekannt, daß gewisse biologische Wirkungen erst durch Zusammenspiel von mindestens zwei Liganden (im biologischen, nicht im erweiterten Sinn nach vorliegender Erfindung) die erwünschte biologische Wirkung, z.B. eine Zellaktivierung, hervorrufen. Dies ist z.B. bei der Kombination gewisser Interleukine in bezug auf deren Wirkung als T- oder B-Zellaktivatoren wünschenswert. Erfindungsgemäß können derartige erst in Kombination wirksame Effektoren in einem erfindungsgemäßen Komplex angeordnet sein. Allerdings ist auch denkbar, daß Zusammensetzungen bereitgestellt werden, die bspw. in bezug auf ihre jeweilige Komponente A verschiedenartige Oligomere enthalten.

In einer weiteren bevorzugten Ausführungsform handelt es sich bei der Komponente A im rekombinanten Fusionsprotein um ein Peptidhormon, einen Wachstumsfaktor, ein Cytokin, ein Interleukin oder einen Abschnitt derselben, vorzugsweise um einen bindungsfähigen Abschnitt. Aber auch funktionelle Derivate der vorgenannten Peptide und/oder Proteine können als Komponente A im rekombinanten Fusionsprotein, das Bestandteil eines erfindungsgemäßen Oligomers ist, zum Einsatz kommen.

Als funktionelle Derivate von biologisch aktiven Proteinen, Proteinabschnitten oder Peptiden werden insbesondere solche Proteine bezeichnet, die die biologische Funktion aufrechterhalten, gleichwohl aber Sequenzunterschiede zu den entsprechenden nativen Sequenzen aufweisen. Bei diesen Sequenzabweichungen kann es sich um eine oder mehr Insertion(en), Deletion(en) oder Substitution(en) handeln, wobei eine Sequenzhomologie von mindestens 70% bevorzugt und eine Sequenzhomologie von mindestens 85 % zwischen dem eingesetzten Derivat und der nativen Sequenz ganz besonders bevorzugt ist. Insbesondere fallen unter den Begriff der funktionellen Derivate solche Aminosäuresequenzen, die gegenüber den physiologischen Sequenzen konservative Substitution aufweisen. Als konservative Substitutionen werden solche Substitutionen bezeichnet, bei denen Aminosäuren gegeneinander ausgetauscht werden, die aus der gleichen Klasse stammen. Insbesondere gibt es Aminosäuren mit aliphatischen Seitenketten, positiv oder negativ geladenen Seitenketten, aromatischen Gruppen in der Seitenketten oder Aminosäuren, deren Seitenketten Wasserstoffbrücken eingehen können, bspw. Seitenketten, die eine Hydroxyfunktion besitzen. Das bedeutet, daß bspw. eine Aminosäure mit einer polaren Seitenkette durch eine andere Aminosäure mit einer gleichfalls polaren Seitenkette ersetzt wird oder beispielsweise eine durch eine hydrophobe Seitenkette gekennzeichnete Aminosäure durch eine andere Aminosäure mit gleichfalls hydrophober Seitenkette substituiert wird (z.B. Serin (Threonin) durch Threonin (Serin) bzw. Leucin (Isoleucin) durch Isoleucin (Leucin)).

Unter einem Liganden werden i.S. der vorliegenden Erfindung alle an Bindungsreaktionen beteiligten Moleküle verstanden. Bei einem Liganden kann es sich demnach auch um ein normalerweise als Rezeptor bezeichnetes Protein handeln. Auch ein solcher Rezeptor kann "Ligand" i.S. dieser Erfindung sein, wenn er an ein Signalmolekül bindet.

Erfindungsgemäß bevorzugt sind Oligomere von Trimeren von rekombinanten Fusionsproteinen, insbesondere Tri- oder Quattromere von Trimeren (3 x 3 oder 4 x 3) oder Hexamere von Trimeren (6 x 3).

Besonders bevorzugt ist ein Bi- oder Oligomer eines Di-, Tri-, Quattro- oder Pentamers von rekombinanten Fusionsproteinen dann, wenn die Komponente A im rekombinanten Fusionsprotein ein Cytokin aus der Familie TNF-Cytokine, ein Abschnitt eines solchen TNF-Cytokins oder ein funktionelles Derivat eines TNF-Cytokins oder eines entsprechenden TNF-Cytokin-Abschnitts ist. Dabei können die eingesetzten TNF-Cytokine bei den Zielzellen durch Bindung an die entsprechenden Rezeptoren bspw. apoptotische, proliferative oder aktivierende Wirkungen hervorrufen. In einer nicht abschließenden Aufzählung kommen als TNF-Cytokine insbesondere die Proteine CD40L, FasL, TRAIL, TNF, CD30L, OX40L, RANKL, TWEAK, Lta, Ltab2, LIGHT, CD27L, 41-BB, GITRL, APRIL, EDA, VEGI und BAFF in Betracht. Als Komponente A im rekombinanten Fusionsprotein werden dabei bevorzugt extrazelluläre Abschnitte der vorgenannten membranständigen TNF-Cytokine oder deren funktionelle Derivate verwendet. Ganz besonders bevorzugt sind diese Spaltprodukte dann, wenn deren jeweilige biologische Funktionalität, insbesondere deren Bindungsdungsvermögen an den jeweiligen Rezeptor, erhalten bleibt. Auch funktionelle Derivate, im oben genannten Sinn, der vorgenannten TNF-Cytokine oder Abschnitte der TNF-Cytokine können als Komponente A des Fusionsproteins zum Einsatz kommen. In einer ganz besonders bevorzugten Ausführungsform ist die Komponente A des rekombinanten Fusionsproteins ausgewählt aus der Gruppe, bestehend aus hFasL (AA 139-261), hTRAIL (AA 95-281), hCD40L (AA 116-261) und m oder hTNFα (AA 77-235).

Aber auch Rezeptoren (membranständige oder intrazelluläre Rezeptoren), insbesondere Rezeptoren von Proteinen der Familie der TNF-Cytokine, insbesondere die physiologischen Rezeptoren der vorgenannten TNF-Cytokine, bzw. Abschnitte oder Derivate der Rezeptoren, werden in einer bevorzugten Ausführungsform als Komponente A im rekombinanten Fusionsprotein eingesetzt. Für den Fall, daß Abschnitte von Rezeptoren als Komponente A eingesetzt werden, wird es sich insbesondere um Abschnitte der physiologischen Proteinsequenz derartiger Rezeptoren handeln, die physiologisch extramembranös angeordnet sind. Hier kommen ganz besonders die extrazellulären Abschnitte derartiger Rezeptoren in Betracht. Beispielsweise kann/können erfindungsgemäß die Bindungsdomäne(n) eines Rezeptors, insbesondere eines Rezeptors, der ein Cytokin aus der Familie der TNF-Cytokine bindet (z.B. FasR, CD30, CD40, GITR, TNF-R1 und/oder TNF-R2), auf einem dimerisierenden Immunglobulin (dimerisierendes Fc-Fragment) aufgetragen sein, und diese Dimere können wiederum durch eine Komponente B, beispielsweise einen Kollagen-artigen Abschnitt mit der Fähigkeit, Di- oder Multimere zu bi- oder oligomerisieren zu erfindungsgemäßen Bi- oder Oligomerkomplexen bi- oder oligomerisiert werden. Hierbei kommt z.B. ein Tetramer von Di- oder Multimeren (z.B. durch tetramerisierende Abschnitte von ACRP30), ein Pentamer von Di- oder Multimeren (z.B durch entsprechende als Komponente B eingesetzte Sequenzabschnitte eines Monomers aus dem COMP-Komplex) oder auch eine Hexamer von Di- oder Multimeren (z.B. durch hexamerisierende Abschnitte aus Monomeren des C1q-Komplexes) in Betracht.

Erfindungsgemäß ergeben sich demnach folgende Möglichkeiten: Die für ein rekombinantes Fusionsprotein, das Bestandteil eines erfindungsgemäßen Oligomers werden soll, ausgewählte Komponente A liegt bereits als solche in Lösung als Di- oder Multimer vor. Die Komponente B wird in einem solchen Fall die Di- oder Multimerisierung der Komponenten A nur noch verstärken und im wesentlichen die Bi- oder Oligomerisierung der rekombinanten Fusionsproteine bewirken. Diese Situation findet sich bspw. dann, wenn als Komponente A mindestens ein TNF-Ligand bzw. ein Abschnitt oder Derivat derselben, die bereits in Lösung typischerweise trimerisiert sind, als Bestandteil(e) eines Fusionsproteins oligomerisiert werden sollen. Für den Fall jedoch, daß die Komponente A als solche in Lösung keine durch Oberflächen-Wechselwirkung vermittelte Di- oder Multimerisierung zeigt, wird die Komponente B erfindungsgemäß sowohl eine Di- oder Multimerisierung der Komponente A als auch eine Bi- oder Oligomerisierung der di- oder multimerisierten rekombinanten Fusionsproteine sicherstellen müssen. Dies ist bspw. typischerweise für den Fall, daß Rezeptoren oder Abschnitte derselben die Komponente A im rekombinanten Fusionsprotein bilden, erforderlich.

Im Rahmen der vorliegenden Erfindung werden auch Bi- oder Oligomere von Di-, Tri-, Quattro- oder Pentameren von rekombinanten Fusionsproteinen offenbart, bei denen vorzugsweise die Komponente A ein Antigen oder ein Abschnitt eines Antigens ist. Erwünscht ist dabei, Antigene von viralen, bakteriellen oder protozoologischen Erregern einzusetzen. Hierbei kann es sich um beliebige typische Antigene eines Erregers, bspw. Proteinabschnitte und/oder spezifische Kohlenhydratstrukturen handeln, typischerweise jedoch um Oberflächenantigene der jeweiligen Erreger oder aber um Abschnitte von Oberflächenproteinen von Erregern, die gleichfalls antigenische Eigenschaften aufweisen. Zu denken wäre hierbei beispielsweise in einer nicht abschließenden Aufzählung an Hämagglutinin, Neuraminidase, PreS1, PreS2, HBs-Antigen, gp120, gp41 oder auch bestimmte typische Tumor-Antigene.

Bei der Komponente A des rekombinanten Fusionsproteins kann es sich aber in einer bevorzugten Ausführungsform auch um eine Aminosäuresequenz handeln, die dazu geeignet ist, als Träger für einen Rezeptoragonisten oder Rezeptorantagonisten zu fungieren. So etwa kann ein als pharmakologischer Wirkstoff aktives, kleines organisch-chemisches Molekül typischerweise kovalent an eine derartige Aminosäuresequenz angekoppelt werden, beispielsweise über eine Etherbindung an Threonin oder Serin, eine amidartige Bindung oder über eine Esterbindung. Durch die vorliegende Erfindung werden dann große Oligomerkomplexe von beispielsweise 18 Fusionsproteinen (z.B. 3 x 6 Fusionsproteinen) mit jeweils angekoppelten Rezeptoragonisten oder Rezeptorantagonisten zur Verfügung gestellt. Hierdurch kann eine erhebliche Verbesserung der Wirksamkeit bzw. der Avidität derartiger organisch-chemischer Moleküle an ihren jeweiligen Rezeptoren, aufgebracht auf einen bi- oder oligomerischen Proteinträger, beispielsweise für den Einsatz als Arzneimittel in der Human- oder Veterinärmedizin, erreicht werden.

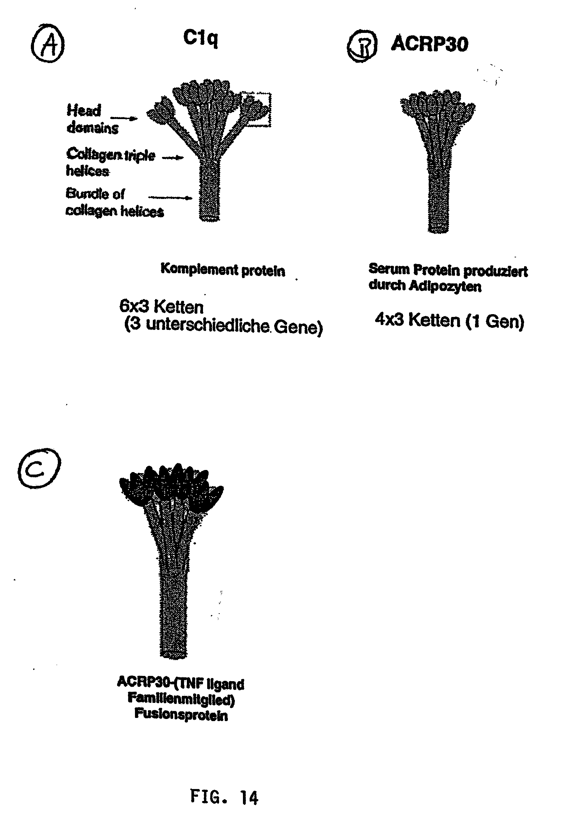

Bei der Komponente B des rekombinanten Fusionsproteins, das di- oder multimerisiert im Bi- oder Oligomer vorliegt, wird es sich typischerweise um ein Protein aus der Familie der Clq-Proteine oder der Collectine handeln. Besonders bevorzugt sind die Proteine der C1q- oder der Collectinfamilie als Bestandteil des rekombinanten Fusionsproteins, nämlich als Komponente B, dann, wenn nur deren Di-/Multimerisierungs- bzw. Bi-/Oligomerisierungssequenz im rekombinanten Fusiosprotein transkribiert bzw. translatiert wird. Die zumeist globulären "Kopf"-Domänen (Figur 14), die in der Sequenz der nativen Monomere enthalten sind, werden also als Translationsprodukt im erfindungsgemäßen rekombinanten Fusionsprotein nicht aufscheinen und sind daher auch nicht Bestandteil der Komponente B in demselben. Die vorgenannte Komponente B in einem erfindungsgemäßen rekombinenten Fusionsprotein wird eine Sequenz aufweisen, die typischerweise zumeist überlappend die Funktionalität zur Di-/Multimerisierung bzw. Bi-/Oligomerisierung aufweist, da die als Komponente B eingesetzten Kollagen-artigen Abschnitte der Proteine der vorgenannten Familien typischerweise an der Bildung von bspw. Tripelhelices beteiligt sind, die wiederum die Fähigkeit besitzen, mit anderen Tripelhelices eine Bi- oder Oligomerstruktur (bspw. ein Tetra-oder Hexamer von bspw. Tripelhelices) einzugehen.

Typischerweise wird daher das multi- und oligomerisierende Fusionsprotein die für die Di- und Multimeriserung bzw. die Bi- und Oligomerisierung verantwortlichen Domänen der Proteine aus den Familien der Clq-Proteine oder Collectine als Komponente B aufweisen, während deren jeweilige "Kopf"-Domänen durch andere, gleichfalls eine biologische Funktion wahrnehmende Proteine oder Proteinabschnitte als Komponente A ersetzt werden. Der Begriff "rekombinantes Fusionsprotein" ist im Rahmen der vorliegenden Erfindung also so zu verstehen, daß die mindestens eine Komponente A und die mindestens eine Komponente B im rekombinanten Fusionsprotein artifiziell fusioniert sind, d.h., daß ein Fusionsprotein i.S. der vorliegenden Erfindung keinem natürlich auftretenden Protein entspricht.

Auch funktionelle, d.h. bi- oder oligomerisierende Derivate von Proteinen aus der Clq-Protein-Familie oder der Familie der Collectine bzw. Derivate von Abschnitten der vorgenannten Proteine können als Komponente B für die Aggregation von rekombinanten Fusionsproteinen zu Bi- oder Oligomeren zum Einsatz kommen. Dabei wird beispielsweise die Komponente B die Sequenz des Proteins C1q, MBP, SP-A ("lung surfactant protein A"), SP-D ("lung surfactant protein D"), BC (Bovines Serumconglutinin), CL43 (Bovines Collectin-43) und/oder ACRP30 bzw. die Sequenz(en) von bi- oder oligomerisierenden Abschnitten mindestens eines der vorgenannten Proteine oder auch von funktionellen Derivaten dieser Proteine oder der Abschnitte der funktionellen Derivate enthalten. Besonders bevorzugt sind Bi- oder Oligomere von rekombinanten Fusionsproteinen dann, wenn die Komponente B des rekombinanten Fusionsproteins ein Proteinabschnitt des Proteins C1q oder des Proteins ACRP30, insbesondere der humanen Variante oder einer anderen Säugetiervariante, ganz besonders der murinen Variante, ist, wobei ein solcher jeweiliger Proteinabschnitt typischerweise keine globuläre "Kopf"-Domäne des nativen Proteins C1q oder ACRP30 aufweist.

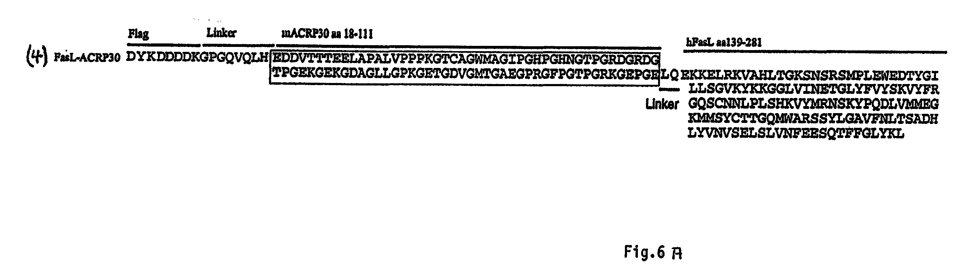

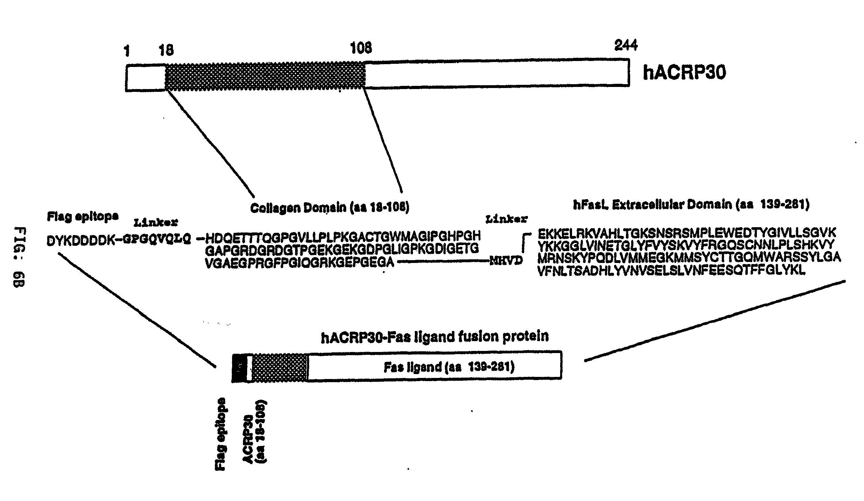

Als ganz besonders bevorzugte Ausführungsform der vorliegenden Erfindung stellen sich solche Bi- oder Oligomere von Di-, Tri-, Quattro- oder Pentameren von rekombinanten Fusionsproteinen dar, deren Komponente B eine Aminosäuresequenz gemäß Figur 6A (eingerahmte Sequenz) bzw. Fig. 6B oder ein funktionelles Derivat dieser Aminosäuresequenz(en) bzw. einen Abschnitt dieser Sequenz(en) enthält. Dabei kann es sich typischerweise um einen Abschnitt des Proteins mACRP30 (m: murin) mit bspw. den Aminosäuren 18 bis 111 oder um einen Abschnitt der humanen Variante (hACRP30) mit bspw. den Aminosäuren 18 bis 108 handeln. Insbesondere kann daher erfindungsgemäß ein Fusionsprotein zur Verfügung gestellt werden, dessen Komponenten A und B humanen Ursprungs sind, so daß etwaige Immunreaktionen beim therapeutischen Einsatz beim Menschen ausgeschlossen werden können.

Insbesondere bevorzugt sind Bi- oder Oligomere von Di- oder Multimeren solcher Fusionsproteine, die Sequenzen aus verschiedenen Wirtsorganismen aufweisen. Ganz besonders bevorzugt sind erfindungsgemäße Aggregate dann, wenn sie aus chimären Fusionsproteinen stammen, wobei die Komponente A aus einer anderen Tierart stammt als die Komponente B. Derart kann es vorteilhaft sein, daß die Komponente A einer Aminosäuresequenz aus der Maus, Ratte, Schwein oder einem anderen Vertebraten, insbesondere aus einem Säuger, oder einem funktionellen Derivat derselben oder entspricht und die Komponente B humanen Ursprungs ist oder umgekehrt. Bevorzugt sind auch erfindungsgemäße Komplexe solcher Fusionsproteine, deren Komponente A einer Sequenz aus einem Virus, Bakterium oder Protozoen entspricht, kombiniert mit einer Komponente B humanen Ursprungs. Selbstverständlich können die Sequenzen der Komponente A und Komponente B in einem erfindungsgemäßen Fusionsprotein aber auch aus der gleichen Tierart stammen.

In einer weiteren bevorzugten Ausführungsform der vorliegenden Erfindung erfolgt die Multi- und/oder Oligomerisierung der Fusionsproteine durch eine kurze Aminosäuresequenz von mehr als 6 Aminosäuren, vorzugsweise von 8 bis 20 Aminosäuren, die in den rekombinanten Fusionsproteinen als Komponente B vorhanden ist. Die durch diese kurzen Aminosäuresequenzen typischerweise erreichte Bi- oder Oligomerisierung von Fusionsproteinen, die bereits als solche durch nicht durch Supersekundärstrukturen, sondern durch Oberflächen-Interaktion in Lösung als Di- oder Multimere vorliegen, beruht bevorzugt auf der Ausbildung von Disulfidbrücken, die durch die spezifische Aminosäuresequenz im rekombinanten Fusionsprotein möglich wird. Dies bedeutet, daß die Komponente B vorzugsweise mindestens ein Cystein aufweist, das dann unter oxidierenden Bedingungen eine kovalente Verknüpfung mit dem mindestens einen Cystein eines Fusionsproteins mindestens eines anderen Di- oder Multimers ausbilden kann. Für den bevorzugten Fall, daß die Komponente B eine kurze bi- oder oligomerisierende Aminosäuresequenz von 8 bis 20 Aminosäuren enthält, wäre als Beispiel die Aminosäuresequenz (Ein-Buchstaben-Code) VDLEGSTSNGRQCAGIRL zu nennen. Diese 18 Aminosäuren umfassende Sequenz weist an Position 11 einen Cystein-Rest auf, der zwischen den Di- oder Multimeren eine Disulfidbrücke bilden kann.

Auch funktionelle Derivate oder Abschnitte dieser 18 Aminosäuren umfassenden Sequenz können als Komponente B zum Einsatz kommen. Hierbei ist insbesondere die Sequenz VDLEGSTSNGRQSAGIRL aufzuführen, bei der zwar an der Position 11 der Cystein- durch einen Serinrest substituiert ist, die aber gleichwohl eine Bi- oder Oligomerisierung der Fusionsprotein-Multimere sicherstellen kann.

Die Fusionsproteine können in einer bevorzugten Ausführungsform auch so ausgestaltet sein, daß über die Bi- oder Oligomerisierung von di- oder multimerisierten Fusionsproteinen hinaus Aggregate höherer Ordnung gebildet werden können. Diese Aggregate höherer Ordnung, die wiederum zwei oder mehr Bi- oder Oligomere umfassen, können beispielsweise durch Vernetzung über Antikörper zur Verfügung gestellt werden. Die Antikörper sind gegen Epitope auf dem (den) Fusionsprotein(en) eines erfindungsgemäßen Komplexes gerichtet, vorzugsweise gegen ein Epitop der Komponente B. Es kann aber auch das Fusionsprotein neben den Komponenten A und B zusätzliche Sequenzabschnitte aufweisen, die als Antigene für die vernetzenden Antikörper dienen. Bevorzugt sind im Rahmen der vorliegenden Erfindung in diesem Zusammenhang sog. Tag-Sequenzen, beispielsweise ein Flag-Tag, also die Aminosäurenfolge DYKDDDDK, oder aber auch beispielsweise ein His-Tag (enthaltend mehrere konsekutive Histidine).

Ganz besonders bevorzugt aber werden im Rahmen der vorliegenden Erfindung Aggregate höherer Ordnung dadurch bereitgestellt, daß mehr als eine Komponente B im rekombinanten Fusionsprotein enthalten ist. Vorzugsweise wird demnach zur Bildung von Aggregaten höherer Ordnung das Fusionsprotein eine Komponente B1 zur Bildung von Bi- oder Oligomeren enthalten und darüber hinaus mindestens eine weitere Komponente B (beispielsweise eine Komponente B2), die typischerweise von der Komponente B1 verschieden ist. Die Komponente B2, die mindestens in einem Fusionsprotein eines erfindungsgemäßen Bi- oder Oligomers auftreten muß, stellt sicher, daß sich die Bi- oder Oligomere zu Aggregaten höherer Ordnung zu vernetzen. Beispielsweise kann es sich bei der Komponente B1 um einen bi- oder oligomerisierenden Abschnitt eines Proteins aus der Familie der C1q- oder Collectin-Proteine, während die Komponente B2 eine mindestens eine Disulfidbrücke bildende 8 bis z.B. 28 Aminosäuren lange Sequenz ist. Für den Fall, daß mindestens eine Disulfidbrücke zwischen zwei verschiedenen Oligomeren gebildet wird, wird ein erfindungsgemäßes Aggregat höherer Ordnung, bspw. ein Bimer eines Oligomers, zur Verfügung gestellt.

Weiterhin wird es bevorzugt sein, zwischen der Komponente A und der(den) Komponente(n) B oder, falls mehrere Komponenten B im Fusionsprotein enthalten sind, auch zwischen diesen mindestens zwei Komponenten B sog. Linkersequenzen einzufügen. Diese Linkersequenzen dienen zur strukturellen Abtrennung der verschiedenen funktionellen Komponenten im rekombinanten Fusionsprotein und können bevorzugt auch eine "Scharnier"-Funktion übernehmen, d.h. eine Aminosäuresequenz flexibler Struktur darstellen.

Damit werden erfindungsgemäß ganz allgemein Bi- oder Oligomere bzw. Aggregate höherer Ordnung offenbart, die sich insbesondere durch eine erhöhte biologische Wirksamkeit und/oder durch erhöhte Aviditäten an komplementäre Proteine auszeichnen. Auf diese Weise werden vorliegend als weiterer Erfindungsgegenstand auch Verfahren offenbart, die zur Erhöhung biologischer Wirksamkeit und/oder zur Erhöhung der Avidität von Biomolekülen oder Pharmaka mit Ligandenfunktion im Sinne der vorliegenden Erfindung dienen. Derartige Verfahren zeichnen sich durch Rekombination mindestens einer Komponente A, die einem Protein oder Proteinabschnitt mit biologischer Funktion entspricht, mit mindestens einer di- oder multimerisierenden und bi- oder oligomerisierenden Komponente B aus, wobei eine Erhöhung der biologischen Aktivität und/oder eine Erhöhung der Avidität der Komponente A erreicht dadurch wird, daß die rekombinierten Fusionsproteine anschließend durch Multi- und Oligomerisierung zu Bi-oder Oligomeren von Di- oder Multimeren bi- oder oligomerisiert werden. Ganz besonders bevorzugt ist das Verfahren dann, wenn die Komponente A ein TNF-Cytokin, ein Abschnitt eines TNF-Cytokins oder ein funktionelles Derivat eines solchen Proteins oder Proteinabschnitts ist. Ein weiteres bevorzugtes Verfahren ist die Rekombination mindestens einer Komponente A mit mindestens einer Komponente B zu einem rekombinanten Fusionsprotein, wobei mindestens eine Komponente A ein Rezeptor oder Abschnitt eines Rezeptors, vorzugsweise eines TNF-Rezeptors, ist. Hinsichtlich bevorzugter Ausführungsformen eines solchen erfindungsgemäßen Verfahrens gelten analog die zuvor beschriebenen bevorzugten Ausführungsformen für die erfindungsgemäßen Bi- oder Oligomere.

Die Bi- oder Oligomere der vorliegenden Erfindung kommen zur Herstellung eines Arzneimittels bzw. zur Behandlung von Erkrankungen oder Störungen zum medizinischen, d.h. sowohl zum human- als auch zum veterinärmedizinischen Einsatz, in Betracht. Auch die aus Bi- oder Oligomeren erfindungsgemäß gebildeten Aggregate höherer Ordnung eignen sich zum Einsatz als Arzneimittel bzw. zur Herstellung eines Arzneimittels. Ein weites Spektrum von Erkrankungen oder Störungen kann mit den erfindungsgemäß beanspruchten Bi- oder Oligomeren bzw. mit den Aggregaten höherer Ordnung behandelt werden. In einer keinesfalls abschließenden Aufzählung können derartige Bi- oder Oligomere bzw. Aggregate höherer Ordnung zur Herstellung eines Arzneimittels zur Behandlung von hyperinflammatorischen Störungen, Autoimmunerkrankungen, Erkrankungen, die auf hyper- oder hypoapoptotischen Reaktionen beruhen, neurodegenerativen Erkrankungen, aber auch zur Behandlung von Infektionskrankheiten, Tumorerkrankungen und/oder endokrinologischen Störungen eingesetzt werden. Unter den Infektionserkrankungen ist die Verabreichung von Bi- oder Oligomeren bei bakteriellen oder protozoologischen Infektionen, insbesondere aber bei viralen Infektionen, zu nennen, wobei als Komponente A im rekombinanten Fusionsprotein dann Antikörper oder Paratope tragende Abschnitte von Antikörpern besonders bevorzugt sind. Bi-oder Oligomere oder deren Aggregate höherer Ordnung sind dann ganz besonders geeignet, wenn die Erkrankung eine Behandlung mit biologisch aktiven Cytokinen erforderlich macht, beispielsweise auch für die Behandlung bzw. die Herstellung eines Arzneimittels zur Behandlung von Tumoren, insbesondere von Tumoren des Lymphsystems.

Weiterhin wird die Verwendung von erfindungsgemäßen Bi-oder Oligomeren als Vakzine bzw. zur Herstellung einer Vakzine zur aktiven oder passiven Immunisierung gegen Infektionskrankheiten zum Einsatz kommen. Hierbei wird als Komponente A im rekombinanten Fusionsprotein typischerweise ein zur Vakzinierung geeignetes Antigen für den Fall der aktiven Immunisierung verwendet werden. Hierfür bieten sich insbesondere Oberflächenantigene oder Abschnitte von Oberflächenantigenen von Bakterien, Viren oder Protozoen, z.B. von Plasmodien oder Trypanosomen, an. In einer nicht abschließenden Aufzählung kann eine Vakzine, die Bi- oder Oligomere oder Aggregate derselben enthält, wobei die rekombinanten Fusionsproteine mindestens eine Komponente A, also ein oder mehr identische oder verschiedene Antigen(e) der Erreger, aufweisen, zur Vakzinierung gegen Röteln, Masern, Polyomyelitis, Tollwut, Tetanus, Diphtherie, BCG, Tuberkulose, Malaria, Gelbfieber, HIV oder Grippeviren, beispielsweise Rhinoviren, eingesetzt werden. Auch die Kombination verschiedener Antigene in einem Bi- oder Oligomer oder einem Aggregat höherer Ordnung aus Bi- oder Oligomeren ist erfindungsgemäß möglich, wobei verschiedene Antigene aus demselben Erreger in einem Bi- oder Oligomer oder aber auch Antigene aus zwei oder mehr Erregern in einem Bi- oder Oligomer oder in einem Aggregat höherer Ordnung kombiniert werden können. Typischerweise können hierfür zwei oder mehr Komponenten A1, A2 bis AX in einem Fusionsprotein enthalten sein, das dann Bestandteil eines erfindungsgemäßen Bi- oder Oligomers oder eines Aggregats höherer Ordnung ist, oder aber zwei oder mehr in bezug auf mindestens eine Komponente A verschiedene Fusionsproteine in einem Bi- oder Oligomer oder Aggregat höherer Ordnung durch mindestens eine Komponente B kombiniert werden.

Bevorzugt können mindestens zwei unterschiedliche erfindungsgemäße Bi- oder Oligomerarten auch in einer Zusammensetzung zur Verwendung als Arzneimittel oder als Vakzine bzw. zu deren Herstellung enthalten sein.

In einer weiteren erfindungsgemäßen Ausführungsform ist die Komponente A in einem erfindungsgemäßen Fusionsprotein ein Immunmodulator, bspw. ein Interleukin (IL), insbesondere IL-2.

Darüber hinaus wird im Rahmen der vorliegenden Erfindung auch die Verwendung von erfindungsgemäßen Bi- oder Oligomeren als Immunisierungs- und/oder Vakzinierungsadjuvans offenbart. Es ist bekannt, daß viele zur Immunisierung verabreichte Antigene beim Probanden eine nur unbefriedigende Immunantwort auslösen. Adjuvantien haben die Aufgabe, die immunogene Wirkung zu erhöhen. Derartige Adjuvantien kommen als Komponente A in einem erfindungsgemäßen Fusionsprotein und damit als Bestandteil eines erfindungsgemäßen Bi- oder Oligomers in Betracht. Bspw. kann die Komponente A eine Aminosäuresequenz aus dem CD40-Liganden (CD40L) oder die Sequenz oder einen Sequenzabschnitt eines Interleukins, bspw. eines der Interleukine 1 bis 12, enthalten. Die physiologische Aufgabe von CD40L ist es, den Übergang einer ruhenden B-Zelle in den Zellzyklus zu steuern. In einem erfindungsgemäßen bi- oder oligomerisierten Komplex können auch Interleukine, bspw. IL-4, IL-5, IL-6, und/oder CD40L kombiniert sein (verschiedene rekombinante Fusionsproteine in einem Bi- oder Oligomer) oder sie können als Komponenten einer Zusammensetzung auftreten (mindestens zwei verschiedene Bi- oder Oligomerarten, die jeweils aus identischen oder unterschiedlichen Fusionsproteinen aufgebaut sein können), die typischerweise neben dem (den) Immunogen(en) mindestens eine, vorzugsweise zwei oder mehr erfindungsgemäße Bi- oder Oligomerarten, enthält.

Die Bi- oder Oligomere oder auch Aggregate höherer Ordnung einer solchen Zusammensetzung können aus in bezug auf die Komponente(n) A identischen oder unterschiedlichen Fusionsproteinen aufgebaut sein. Hierbei kommt als Komponente A jede physiologische Sequenz mit co-stimulierenden und/oder das Immunsystem (zelluläre oder humorale Immunantwort) aktivierenden Eigenschaften in Betracht. Dabei kann es sich um physiologische Verbindungen oder synthetische Verbindungen handeln. Damit werden also auch Zusammensetzungen offenbart, die eine oder mehr erfindungsgemäße Bi- oder Oligomerart(en) neben einem oder mehr Immunogen(en) enthalten, wobei eine erfindungsgemäße Bi- oder Oligomerart vorzugsweise so ausgestaltet sein kann, daß in einem derartigen bi- oder oligomerisierten Komplex mehr als ein Immunmodulator/-adjuvans enthalten ist, also ein Heterobi- oder - oligomer vorliegt. Ggf. kann ein erfindungsgemäßes Heterobi- oder -oligomer auch sowohl ein oder mehr Fusionsproteine mit einem Immunogen als Komponente A als auch mindestens ein Fusionsprotein mit einer Adjuvans-Komponente als Komponente A, bspw. CD40L, vereinen. Auch zwei oder mehr verschiedene Fusionsproteine mit jeweils unterschiedlichen Adjuvans- oder Immunmodulator-Komponenten in einem erfindungsgemäßen Heterooligomer sind denkbar. Damit ist auch die Verwendung derartiger Homo- oder Heteroologimere oder von Zusammensetzungen, die mindestens eine erfindungsgemäße Hetero- oder Homooligomerart enthält, als Arzneimittel oder als Vakzine in der Human- oder Veterinärmedizin offenbart.

Die Bi- oder Oligomere werden im Rahmen der vorliegenden Erfindung bevorzugt so zur Herstellung eines Arzneimittels bzw. zur Behandlung der vorgenannten Erkrankungen oder Störungen verwendet, daß sie zur parenteralen, d.h. beispielsweise subkutanen, intramuskulären oder intravenösen, oder oralen oder intranasalen Verabreichung geeignet sind. Die Verabreichung von Bi- oder Oligomeren oder Aggregaten derselben als Vakzine bzw. als Basis für die Herstellung einer Vakzine wird gleichfalls bevorzugt in einer parenteralen oder oralen Verabreichungsform, ggf. aber auch intranasalen Verabreichungsform, erfolgen.

Die erfindungsgemäßen Bi- oder Oligomere bzw. die Aggregate höherer Ordnung können allein als Arzneimittel dienen bzw. zur Herstellung eines Arzneimittels herangezogen werden. Sie können aber auch in Kombination mit anderen aktiven Wirkstoffkomponenten als Arzneimittel zum Einsatz kommen. Die erfindungsgemäßen Bi- oder Oligomere bzw. die Aggregate höherer Ordnung können aber auch in Kombination mit pharmazeutisch akzeptablen Träger-, Hilfs- und/oder Zusatzstoffen kombiniert werden. Entsprechende Herstellungswege sind bei "Remington's Pharmaceutical Sciences" (Mack Pub. Co., Easton, PA, 1980) offenbart, das Bestandteil der Offenbarung der vorliegenden Erfindung ist. Für die parenterale Verabreichung kommen als Trägerstoffe bspw. steriles Wasser, sterile Kochsalzlösungen, Polyalkylenglykole, hydrogenierte Naphthalen und insbesondere biokompatible Lactidpolymere, Lactid/Glycolidcopolymer oder Polyoxyethylen-/Polyoxypropylencopolymere in Betracht.

Erfindungsgemäße Bi- oder Oligomere oder entsprechende Aggregate höherer Ordnung finden bevorzugt auch Verwendung im Bereich der In-vitro-Diagnose oder aber beispielsweise für biochemische Reinigungsverfahren. Zu denken ist an den Einsatz von Bi- oder Oligomeren bzw. Aggregaten höherer Ordnung derselben auf Reinigungssäulen, die mit derartigen Komplexen bepackt sein können. Damit wird im Rahmen der vorliegenden Erfindung die Verwendung von derartigen Komplexen auch zu Nachweiszwecken offenbart.

Weiterhin werden im Rahmen der vorliegenden Erfindung Verfahren offenbart, um spezifisch assozierte Proteine herzustellen, die auf Grund ihrer Wechselwirkungen an der Proteinoberfläche interagieren und daher als solche in Lösung di- oder multimerisiert vorliegen. Insbesondere bei TNF-Cytokinen oder vorzugsweise löslichen Abschnitten derartiger Cytokine, die in Lösung trimerisieren, ist es erwünscht, diese in einer bestimmten Stöchiometrie in einer reinen Fraktion zur Verfügung zu stellen. Im Falle einer einfachen Koexpression von verschiedenen, untereinander assoziationsfähigen Proteinen, z.B. von drei verschiedenen TNF-Cytokinen oder verschiedenen Abschnitten von derartigen TNF-Cytokinen, finden sich nämlich alle statistisch möglichen Verteilungen der koexprimierten Proteine in den nach der Expression assoziierenden Trimeren, also bspw. die gewünschten Trimere aus den Proteinen P1, P2 und P3, aber auch Trimere aus zwei Proteinen P1 und einem Protein P3 etc.

Erfindungsgemäße Oligomere können nunmehr verfahrensgemäß eingesetzt werden, um bestimmte gewünschte Heteromultimere zu erhalten, bspw. Heterotrimere von TNF-Cytokinen oder Abschnitten derartiger TNF-Cytokine. Hierzu werden verschiedene Fusionsproteine konstruiert und vorzugsweise in einer Wirtszelle exprimiert. Die hier exprimierten Fusionsproteine weisen als Komponente B Sequenzabschnitte von solchen Proteinen auf, die Homooligomere von Heteromultimeren bilden, also bspw. ein Trimer aus drei verschiedenen Ketten bilden, wobei identische Trimere bi- oder oligomerisieren. Vorzugsweise entsprechen diese Komponenten B in den Fusionsproteinen Sequenzabschnitten von Proteinen aus der Komplement- oder Collectinfamilie, bspw. C1q, das Homohexamere von Heterotrimeren bildet. In einem Fusionsprotein ist daher als Komponente B einen Sequenzabschnitt, den das native Protein zur Multimerisierung einer Kette im Heteromultimer vorsieht, mit einer Komponente A, bspw. einem TNF-Cytokin, kombiniert, während weitere Fusionsproteine (die im Heterotrimer auftreten sollen) jeweils Kombinationen einer anderen Komponente A, also bspw. eines anderen TNF-Cytokins oder eines Abschnitts hiervon, mit einem jeweils anderen im nativen Protein, bspw. C1q-Protein, vorhandenen Sequenzabschnitt zur Heteromultimerisierung aufweisen.

Vorzugsweise in einer Wirtszelle werden nunmehr die verschiedenen Fusionsproteine, die das Heteromultimer bilden können, exprimiert. Die Heteromultimere lagern sich dann zu Homooligomeren zusammen, da die Komponente B nur identische Heteromultimere oligomerisieren kann. Erfindungsgemäß können bspw. drei verschiedene Fusionsproteine mit jeweils unterschiedlichen Komponenten A, also vorzugsweise unterschiedlichen TNF-Cytokinen, die jeweils entweder mit der multi- und oligomerisierenden α-, β- oder γ-Kette von C1q kombiniert werden, exprimiert werden. In den assoziierenden Oligomeren finden sich dann ausschließlich Heteromultimere, die alle drei TNF-Cytokine aufweisen. Heteromultimere mit anderer Stöchiometrie treten dagegen nicht auf. Allein durch die Wahl der Fusionsproteine läßt sich damit erfindungsgemäß ein in seiner Stöchiometrie spezifisches und nicht einer statistischen Verteilung unterliegendes Produkt erhalten.

Bevorzugt ist darüber hinaus der Einsatz solcher Fusionsproteine bzw. Verfahren zur Gewinnung von Heteromultimeren vorgegebener Stöchiometrie, die einen Linker zwischen der Komponente A und der Komponente B aufweisen. Ganz besonders bevozugt sind die Linker dann, wenn sie mindestens eine proteolytische Schnittstelle enthalten, die es erlaubt, die Komponenten A aus dem Homooligomerkomplex (von Heteromultimeren) von den Komponenten B abzutrennen. Damit wird schließlich eine Fraktion erhalten, die ausschließlich aus dem gewünschten Heteromultimer, bspw. einem Heterotrimer aus drei verschiedenen TNF-Cytokinen, besteht. Bei der proteolytischen Schnittstelle im Linker handelt es sich vorzugsweise um eine Thrombin-Konsensussequenz.

Als weiterer Gegenstand der vorliegenden Erfindung werden vorliegend Fusionsproteine beschrieben, die zur Bi- bzw. Oligomerisierung von Di- oder Multimeren geeignet sind, sofern das rekombinante Fusionsprotein mindestens eine Komponente A und mindestens eine Komponente B enthält, wobei die Komponente A ein Protein oder einen Proteinabschnitt mit biologischer Funktion, insbesondere mit Ligandenfunktion für Antikörper oder Rezeptoren oder aber einen Antikörper oder Abschnitt eines Antikörpers enthält, und die Komponente B einen di- oder multimerisierenden und bi- oder oligomerisierenden Abschnitt oder ein funktionelles Derivat eines solchen Abschnitts eines Proteins, ausgewählt aus der Gruppe, bestehend aus der Familie der C1q-Proteine oder der Collectine, enthält. Ganz besonders bevorzugt sind derartige Fusionsproteine dann, wenn die Komponente B des rekombinanten Fusionsproteins einen multi- und/oder oligomerisierenden Abschnitt des Proteins C1q, MBP, SP-A, SP-D, BC, CL43 und ACRP30 enthält. Auch ein funktionelles Derivat eines solchen Abschnitts der vorgenannten Proteine kann im Rahmen der vorliegenden Erfindung zum Einsatz kommen. Ein Fusionsprotein wird in diesem Fall neben einer mit biologischer Aktivität ausgestatteten Komponente A typischerweise eine Komponente B enthalten, die ausschließlich den für die Aggregation verantwortlichen Abschnitt der vorgenannten Proteine aufweist, vorzugsweise nicht aber die globuläre "Kopf"-Domäne derselben.

Als Komponente B eines erfindungsgemäßen Fusionsproteins kommt vorzugsweise auch eine Sequenz in Betracht, die die Aminosäuresequenz der oligomerisierenden Kollagendomäne des Liganden EDA, insbesondere die Variante eines Säugetiers, ganz besonders die humane Variante, oder eines oligomerisierenden Fragments oder funktionellen Derivats einer solchen Domäne enthält. Ganz besonders bevorzugt wird als Komponente B ein Sequenzabschnitt, der die Aminosäuren 160 bis 242 des humanen Proteins EDA oder eines funktionellen Derivats, bspw. eines Fragments, enthält, zum Einsatz kommen. Vorzugsweise wird es sich um ein Hexamer handeln.

Ein weiterer Gegenstand der vorliegenden Erfindung sind DNA-Sequenzen, die für Fusionsproteine der vorgenannten Art kodieren. Derartige DNA-Sequenzen werden in Expressionsvektoren exprimiert, wobei auch die entsprechenden Expressionsvektoren, die eine DNA-Sequenz für die erfindungsgemäßen Fusionsproteine enthalten, Gegenstand der Erfindung sind. Weiterhin gehören zur vorliegenden Erfindung solche Wirtszellen, die mit DNA-Sequenzen, die für die erfindungsgemäßen Fusionsproteine kodieren, transfiziert sind. Ganz besonders bevorzugt sind in diesem Zusammenhang Wirtszellen, die mit Expressionsvektoren transfiziert sind, wobei die Expressionsvektoren wiederum DNA-Sequenzen enthalten, die für die erfindungsgemäßen Fusionsproteine kodieren.

Ein weiterer Gegenstand der vorliegenden Erfindung sind Rezeptoren, insbesondere Rezeptoren, die Signalmolekül-Liganden aus der TNF-Cytokinfamilie binden, die di- oder multimerisiert vorliegen. So etwa kann ein derartiger Rezeptor, ein Derivat desselben oder ein Abschnitt eines Rezeptors oder Derivats, insbesondere ein Abschnitt, der den extrazellulären Bereich des Rezeptors umfaßt, wobei wiederum die Bindungsdomäne(n) bevorzugt sind, als Komponente A eines Fusionsproteins, das Betandteil eines Di- oder Multimers ist, vorliegen. Die Dimerisierung kann durch Rekombination mit Abschnitten von Immunglobulinen, insbesondere Fc-Fragmenten, erreicht werden, während die Multimerisierung, z.B. nach Rekombination mit den entsprechenden Multimerisierungsdomänen von Proteinen, erreicht werden kann. Hierzu sind bspw. alle Sequenzabschnitte von Proteinen geeignet, die durch Ausbildung von Supersekundärstrukturen Di- oder Multimere generieren, z.B. "Coiled-Coil-Helices" oder typische Kollagen-artige Tripelhelices (z.B. CMP, COMP, Kollagen, Laminin). Auch Abschnitte von Proteinen aus der C1q-Familie oder von Collectinen sind typischerweise für eine Di- oder Multmerisierung von Rezeptoren oder Rezeptorabschnitten geeignet. So etwa kann bspw. der extrazelluläre Abschnitt eines Mitglieds aus der TNF-Rezeptorfamilie, bspw. der Fas-Rezeptorr (FasR), als Komponente A in Form eines Pentamers durch Rekombination mit den entsprechenden Pentamerisierungsdomänen von COMP als Komponente B exprimiert werden. Dabei kann es sich um Homo- oder Heterodi- oder -multimere von Fusionsproteinen, die einen Rezeptor oder Rezeptorabschnitt aufweisen, handeln.

Diese Di- oder Multimere eines rekombinaten Proteins mit einer einen Rezeptor oder Rezeptorabschnitt enthaltenden Komponente A kommen auch als Arzneimittel oder zur Herstellung eines Arzneimittels in Betracht. Ihre Verwendung ist insbesondere dann gegeben, wenn erhöhte extrazelluläre Konzentrationen der entsprechenden Rezeptor-Liganden bei einem Krankheitsbild auftreten. Hierbei kann es sich auch um erhöhte Konzentrationen von membranständigen Signalmolekülen, bspw. TNF-Cytokinen, auf den Zellen selbst oder von löslichen Signalmolekülen handeln. Die Verwendung derartiger Multimere ist aber grundsätzlich auch immer dann gewünscht, wenn die Aktivierung einer Signaltransduktionskette, die an dem entsprechenden typischerweise membranständigen Rezeptor ausgelöst wird, durch den Einsatz von die Signalmoleküle wegbindenden, exogenen löslichen erfindungsgemäßen Di- oder Multimeren, bestehend aus Fusionsproteinen, die als Komponente A einen Rezeptor oder Rezeptorabschnitt enthalten, verhindert oder herabgesetzt werden soll.

Die vorliegende Erfindung wird durch die nachfolgenden Figuren näher erläutert:

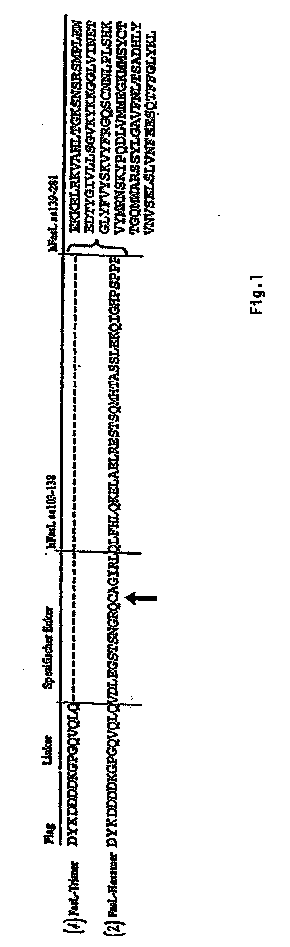

In Figur 1 ist im Ein-Buchstaben-Code die Aminosäuresequenz eines in einem erfindungsgemäßen Oligomer (FasL-Hexamer), also im Bimer eines Trimers, auftretenden rekombinanten erfindungsgemäßen Fusionsproteins (2) dargestellt. Als Komponente A wird ein Sequenzabschnitt des hFasL (AS 103 bzw. 139 bis 281) bezeichnet, während als Komponente B der spezifische (im Ergebnis bimerisierende) Linker mit der Sequenz VDLEGSTSNGRQCAGIRL auftritt. Weiterhin findet sich in Figur 1 die Aminosäuresequenz des rekombinanten Fusionsproteins (1), das entsprechend dem Stand der Technik nur als Bestandteil eines FasL-Trimers auftritt, also keine das vorliegende Multimer, hier Trimer, bi- oder oligomerisierende Komponente B aufweist. Die rekombinierten Abschnitte der beiden Fusionsproteinen (1) und (2) sind in der Figur 1 oberhalb der Sequenzangaben in bezug auf ihre jeweilige Funktionalität gekennzeichnet.

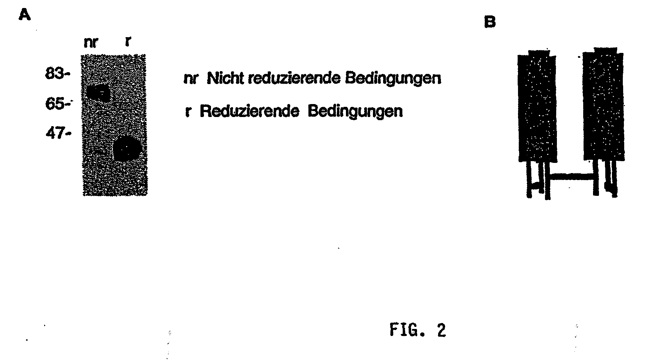

Figur 2 umfaßt in Figur 2A das Ergebnis einer Gelelektrophorese (SDS-PAGE) von erfindungsgemäßen Fusionsproteinen (2), also mit der spezifischen Linker-Sequenz (Komponente B), unter reduzierenden (r) und nicht-reduzierenden Bedingungen (nr). Unter nicht-reduzierenden Bedingungen in Lösung eluiert erfindungsgemäß das Oligomer als nativer Komplex aus sechs erfindungsgemäßen Polypeptiden (Fusionsproteinen (2)), da es erfindungsgemäß zur Ausbildung einer Disulfidbrücke zwischen den Komponenten B von zwei erfindungsgemäßen Fusionsproteinen (2), die jeweils Bestandteil verschiedener Trimere sind, kommt. Im Ergebnis liegt daher ein erfindungsgemäßes Oligomer als FasL-Hexamer mit einem Molekulargewicht vor, das ca. dem 6-fachen Molekulargewicht des erfindungsgemäßen Fusionsproteins (2) in monomerischer Form entspricht. Wenn denaturierende Bedingungen gegeben sind (z.B. auf SDS-PAGE) wandern die erfindungsgemäßen Fusionsproteine in Abwesenheit von Reduktionsmitteln als Dimere, bedingt durch die die Ausbildung einer Disulfidbrücke zwischen zwei Monomeren. Unter reduzierenden und denaturierenden Bedingungen wandern dagegen Monomere des erfindungsgemäßen Fusionsproteins (2) im SDS-Gel. Schematisch ist ein erfindungsgemäßes Oligomer, hier ein Bimer eines Trimers, eines erfindungsgemäßen Fusionsproteins, bspw. eines Fusionsproteins (2), wie in Figur (1) gezeigt, in Figur 2B dargestellt.

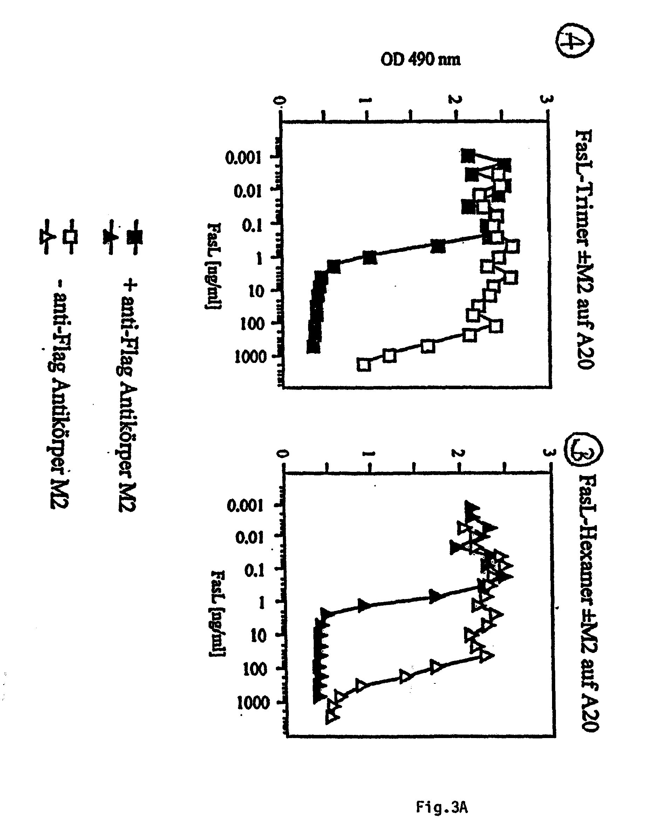

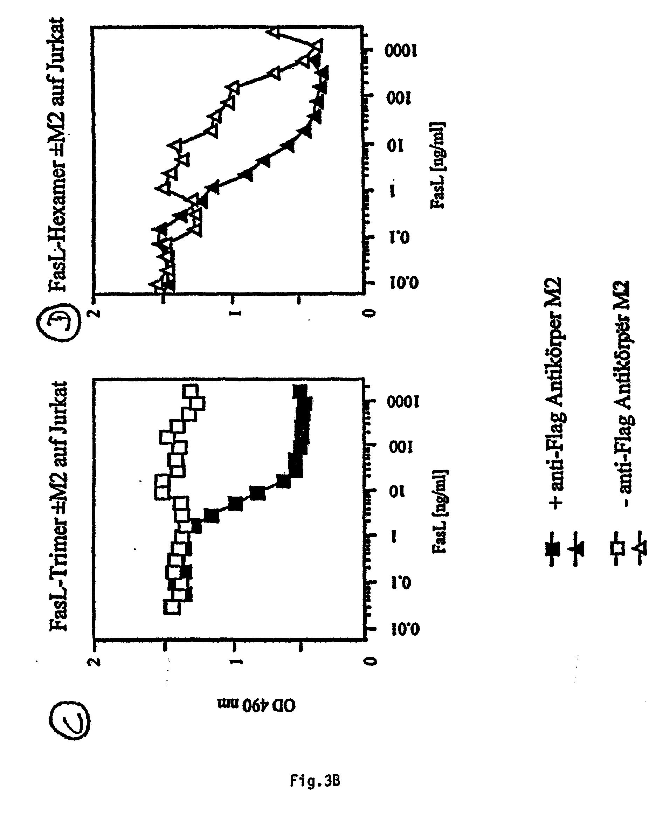

In Figur 3 ist das Ergebnis der zytotoxischen Assays in Abhängigkeit von der Konzentrationen von FasL-Trimeren (Trimer aus drei Fusionsproteinen nach dem Stand der Technik, bspw. Fusionsprotein (1) gemäß Fig. 1, die keine Komponente B aufweisen) oder des in Figur 2B schematisch abgebildeten erfindungsgemäßen FasL-Bimers (Hexamer als Bimer von Trimeren) in Gegenwart (■,▲) oder Abwesenheit (□, Δ) von Anti-Flag-Antikörper M2 für A20- bzw. Jurkat-Zellen dargestellt. Die optische Dichte bei 490 nm ist ein Maß für die Lebensfähigkeit der Zellen (hohe optische Dichte entspricht einer geringen apoptotischer Wirkung der zugegebenen Substanzen und damit einer hohen Lebensfähigkeit der Zellen) . Auf A20-Zellen (Figur 3A und 3B) und auf Jurkat-Zellen (Figur 3C und 3D) ist die apoptotische Wirkung von erfindungsgemäßen FasL-Bimeren von Trimeren (Hexameren) (Figur 3B und 3D, jeweils Δ) gegenüber der Wirkung der FasL-Trimere von Fusionsproteinen ohne eine bi- oder oligomerisierende Komponente B (Stand der Technik, Figur 3A und 3C, jeweils □) 3- bis 10-fach erhöht. Bei zusätzlicher Zugabe von Anti-Flag-Antikörpern, die gegen die Flag-Sequenz der Fusionsproteine (1) und (2) gemäß Fig. 1 gerichtet sind und durch Vernetzung bspw. auch den Oligomerisierungsgrad der erfindungsgemäßen Fusionsproteine weiter erhöhen, wird die apoptotische Aktivität in allen Ansätzen (Figuren 3A bis 3D, jeweils ■ oder ▲) gesteigert.

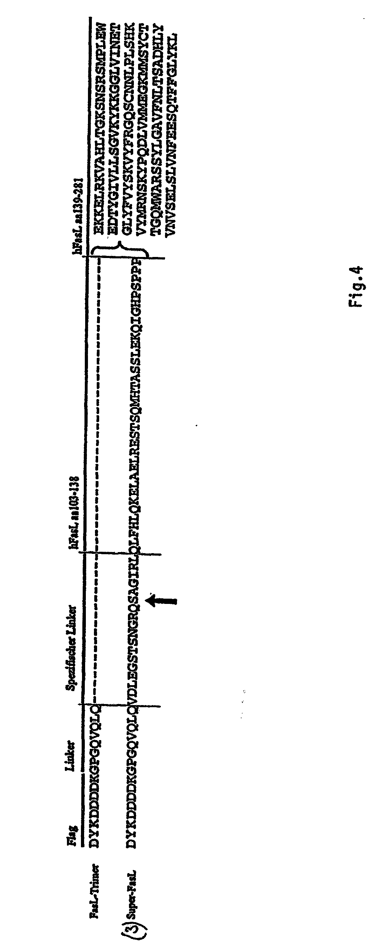

In Figur 4 ist die Aminosäuresequenz eines erfindungsgemäßen Fusionsproteins (3) unter Berücksichtigung der (C→S)-Substitution im spezifischen Linkerbereich (Komponente B von Fusionsprotein (3)) dargestellt ("Super-FasL"). Im übrigen wird auf die Beschreibung zu Fig. 1 verwiesen. Durch Gelfiltrationsexperimente wurde gezeigt, daß "Super-FasL" in Lösung in einer erfindungsgemäß bimerisierten Form als Hexamer vorliegt. Unter denaturierenden Bedingungen auf SDS-PAGE wandern erfindungsgemäße Fusionsproteine (3), auch in Abwesenheit von Reduktionsmitteln, als Monomere, da sich im Linker-Bereich auf Grund der Aminosäuresubstitution C→S keine Disulfidbrücke bilden kann.

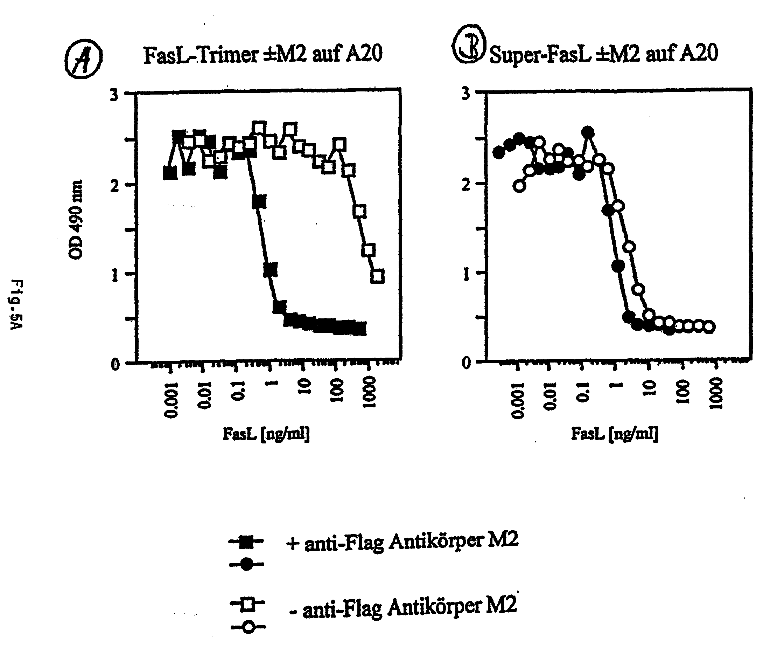

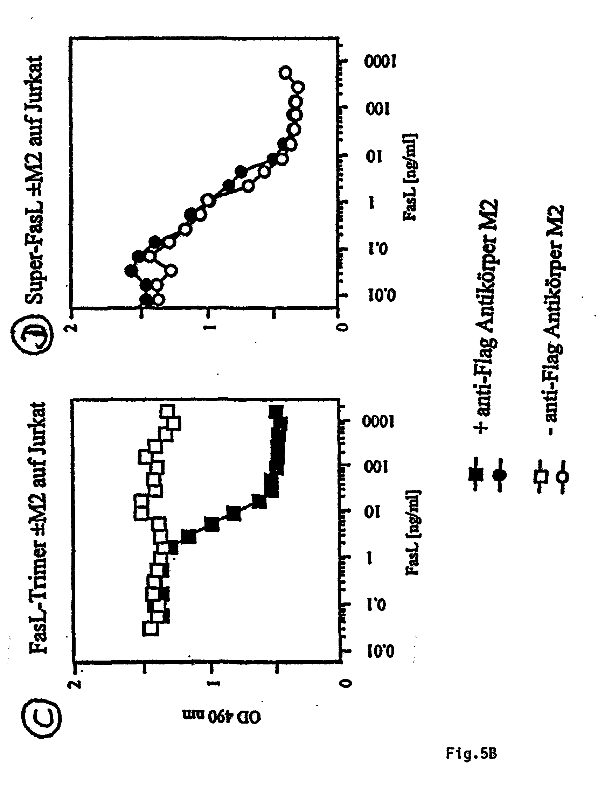

Figur 5 zeigt analog zu Figur 3 die Lebensfähigkeit von A20- (Figur 5B) oder Jurkat-Zellen (Figur 5D) nach Zugabe von erfindungsgemäßen Aggregaten des erfindungsgemäßen Fusionsproteins (3) gemäß Fig. 4 ("Super-FasL") in Gegenwart (•) oder Abwesenheit (o) von Anti-Flag-Antikörper M2. Die erfindungsgemäßen Fusionsproteine (3) rufen eine gegenüber den zum Vergleich eingesetzten FasL-Trimeren nach dem Stand der Technik ohne bi- oder oligomerisierende Komponente B (Figur 3A (A20-Zellen) und Figur 3C (Jurkat-Zellen)) eine ungefähr mindestens 1000-fach verstärkte apoptotische Wirkung hervor. Die Zugabe von Anti-Flag-Antikörpern M2 (•) vermag durch weitergehende Oligomerisierung von "Super-FasL" zu erfindungsgemäßen Aggregaten höherer Ordnung sowohl auf A20- als auch auf Jurkat-Zellen die apoptotische Aktivität gegenüber dem Ansatz ohne Anti-Flag-Antikörper geringfügig, ca. 2-fach, vorzugsweise mindestens 1,5-fach, zu steigern (Figuren 5B und 5D). Daraus ergibt sich, daß der durch den als Komponente B verwendeten spezifischen Linker eines erfindungsgemäßen Fusionsproteins (3) bewirkte Oligomerisierungsgrad (hier als Bimer von Trimeren) für die Apoptoseauslösung, ggf. durch Oligomerisierung auf der Zelloberfläche, bereits nahezu optimal ist und durch erfindungsgemäße Aggregate höherer Ordnung noch geringfügig gesteigert werden kann.
In Figur 6A ist die Aminosäuresequenz eines erfindungsgemäßen Fusionsproteins (4), FasL-ACRP30, gezeigt, wobei das Fusionsprotein (4) (in der Abfolge vom N- zum C-Terminus) eine Flag-Sequenz, eine Linkersequenz als Komponente B, die Aminosäuren 18 bis 111 vom Protein mACRP30 (m: murin), den Linker LQ und dann die Aminosäuren 139 bis 281 von hFasL als Komponente A aufweist.
In Figur 6B ist ein weiteres Fusionsprotein dargestellt. Es umfaßt die Kollagen-Domäne des humanen ACRP30-Liganden (hACRP30, mit den Aminosäuren 18 bis 111) , wobei dessen N-Terminus an eine Flag-Markierung (DYKDDDDK) und dessen C-Terminus an die extrazelluläre Domäne von humanem FasL (AS 139 bis 281) fusioniert ist. Zwischen dem C-Terminus von hACRP30 und hFasL ist eine Linker-Sequenz (MHVD) ebenso eingefügt wie zwischen der Flag-Markierung und dem N-Terminus von hACRP30 (GPGQVQLQ). Alle vorstehenden Angaben beziehen sich auf den sog. Ein-Buchstaben-Code der Aminosäuren.

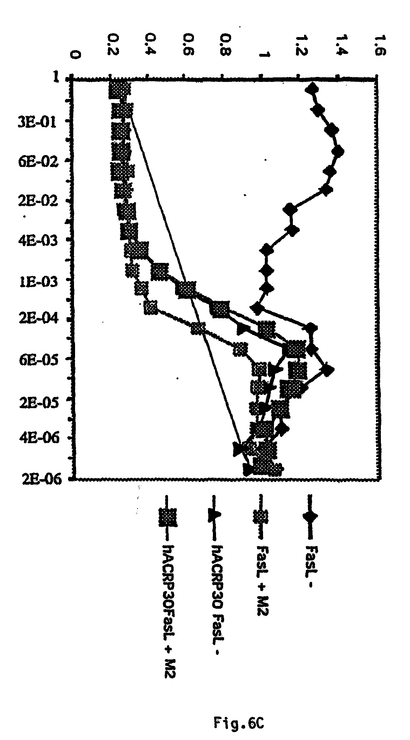

Figur 6C stellt Kurvenverläufe dar, die sich aus der Titration von Jurkat-T-Zellen mit einerseits FasL und andererseits dem Fusionsprotein hACRP30/FasL unter Zugabe von Anti-Flag-Antikörper M2 (+) und ohne Zugabe von Anti-Flag-Antikörper M2 (-) ergeben. Hierzu wurden Überstände (OPTIMEM) von transient mit FasL oder hACRP30/FasL transfizierten und diese Proteine exprimierenden 293-Zellen zu Jurkat-T-Zellen gegeben. In aufeinanderfolgenden Experimenten wurde hierzu die auf der x-Achse von Fig. 6C dargestellten, abnehmenden Konzentrationen eingesetzt und die jeweilige Überlebensrate der Jurkat-Zellen mit dem an anderer Stelle beschriebenen Standard-Zytotoxizitätstest bestimmt. Aus der Auftragung wird deutlich, daß FasL ohne kreuzvernetzenden M2-Antikörper inaktiv ist (◆) und erst die kreuzvernetzende Wirkung des Antikörpers M2 zytotoxische Effekte hervorruft. Demgegenüber wird die entsprechende Wirkung von einem erfindungsgemäßen Fusionsprotein, nämlich hACRP30/FasL, bereits ohne Zugabe von M2-Antikörper gezeigt (▲). Die Zugabe von M2-Antikörpern kann die Wirkung des erfindungsgemäßen Fusionsproteins kaum steigern.

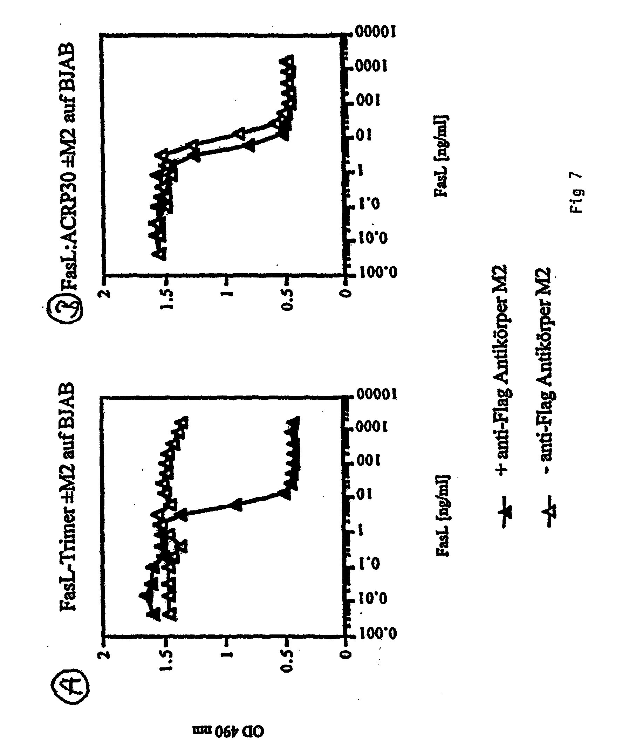

Figur 7 zeigt analog zu Figur 3 die Lebensfähigkeit von BJAB-Zellen nach Zugabe von nicht bi- oder oligomerisierungsfähigen FasL-Trimeren (Vergleichsversuch, auf der Basis des Fusionsproteins (1) gemäß Figur 1; Figur 7A) und von erfindungsgemäßen Oligomeren, hier als Tetramer von Trimeren, also Dodekameren, des erfindungsgemäßen Fusionsproteins (4) gemäß Fig. 6A in Gegenwart (▲) oder Abwesenheit (Δ) von Anti-Flag-Antikörper M2. Während die nichterfindungsgemäßen FasL-Trimere in Abwesenheit von durch Bindung an die Flag-Sequenz oligomerisierenden Antikörpern keine apoptotische Wirkung auf BJAB-Zellen entfalten (Figur 7A, (Δ)), induziert ein erfindungsgemäßes Dodekamer (Tetramer von Trimeren)) von Fusionsprotein (4) bereits bei einer Konzentration von ca. 10 ng/ml (K₅₀= 8 ng/ml) den Zelltod (Figur 7B). Dies manifestiert sich deutlich durch eine Verringerung der OD bei 490 nm. Diese apoptotische Aktivität kann durch Zugabe von Anti-Flag-Antikörpern M2 zu einem Ansatz von erfindungsgemäßen Dodekameren, bestehend aus erfindungsgemäßen FasL-ACRP30-Fusionsproteinen, geringfügig erhöht werden, d.h. ein erfindungsgemäßes Dodekamer stellt einen in Hinblick auf die apoptotische Aktivität bereits annähernd optimalen Aggregationszustand dar. Die durch entsprechende Antikörper ausgelöste weitere Aggregation zu erfindungsgemäßen Aggregaten höherer Ordnung vermag dagegen keine weiteren signifikanten biologischen Effekte hervorzurufen.

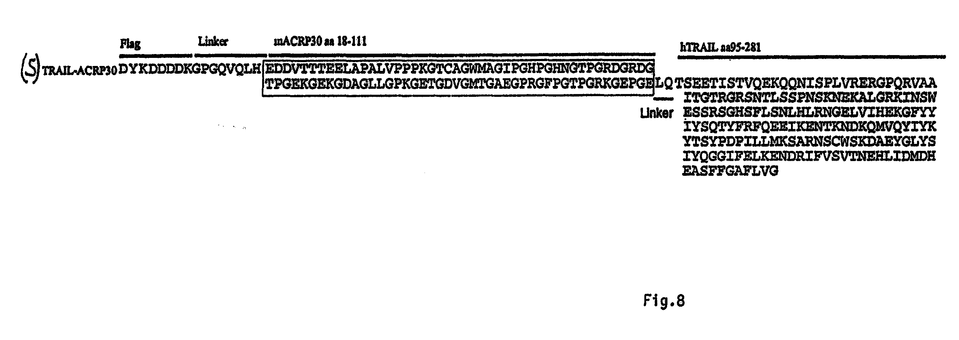

Die Aminosäuresequenz eines erfindungsgemäßen Fusionsproteins (5), das die Aminosäuren 95 bis 281 des hTRAIL als Komponente A in Kombination mit der Oligomerisierungsdomäne von ACRP30 (AS 18-111) als Komponente B enthält, ist in Figur 8 gezeigt (TRAIL-ACRP30). Erfindungsgemäß liegt daher das Fusionsprotein (5) in Lösung als Oligomer, nämlich als Tetramer von Trimeren, vor.

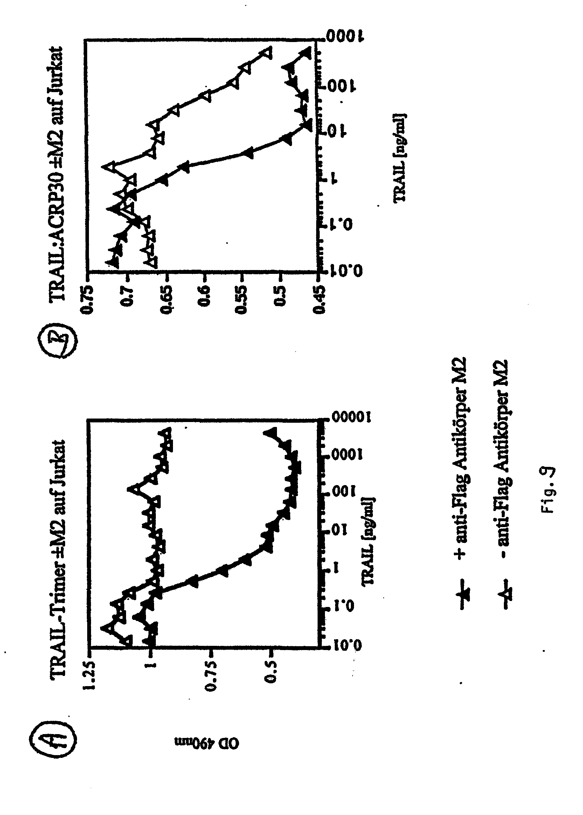

Figur 9 gibt die Lebensfähigkeit von Jurkat-Zellen nach Zugabe von TRAIL-Trimeren (Fusionsprotein mit einer Flag-Sequenz am N-Terminus und den Aminosäuren 95 bis 281 von humanem TRAIL) nach dem Stand der Technik ohne Bi- oder Oligomerisierungsfähigkeit (Vergleichsversuch, Figur 9A) und von erfindungsgemäßen Dodekameren des erfindungsgemäßen. Fusionsproteins (5), TRAIL-ACRP30, in Gegenwart (▲) oder Abwesenheit (Δ) von Anti-Flag-Antikörper M2 wieder. Die experimentellen Beobachtungen in Figur 9 entsprechen den in Figur 7 abgebildeten Ergebnissen. Während die TRAIL-Trimere in Abwesenheit von durch Bindung an die Flag-Sequenz oligomerisierenden Antikörpern keine apoptotische Wirkung auf die Jurkat-Zellen entfalten (Figur 9A, (Δ)), induziert das erfindungsgemäße Dodekamer von Fusionsprotein (5) auch in diesem Experiment bereits bei einer Konzentration von ca. 100 ng/ml (≈K₅₀) den Zelltod (Figur 9B, (Δ)). Die kombinierte Zugabe von TRAIL-Dodekameren und Anti-Flag-Antikörpern kann darüber hinaus aus den erfindungsgemäßen Oligomeren durch weitere Erhöhung des Oligomerisierungsgrads erfindungsgemäße Aggregate höherer Ordnung derselben bilden, die in diesem Fall eine weiter (mindestens 10-fach) gesteigerte Apoptose induzierende Aktivität haben (Figur 9B, (▲)).

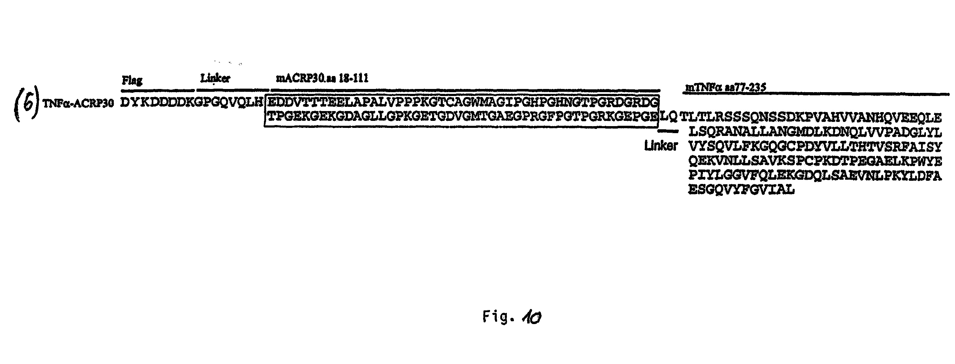

Die Aminosäuresequenz eines erfindungsgemäßen Fusionsproteins (6), das die Aminosäuren 77 bis 235 des mTNFα (m: murin) als Komponente A in Kombination mit der Oligomerisierungsdomäne von mACRP30 (AS 18-111) als Komponente B enthält und eine Flag-Sequenz mit Linker am N-Terminus aufweist, ist in Figur 10 gezeigt (TNFα-ACRP30). Damit liegt das Fusionsprotein (6) in Lösung als Dodekamer, nämlich als Oligomer (Tetramer) von Multimeren (Trimeren), vor.

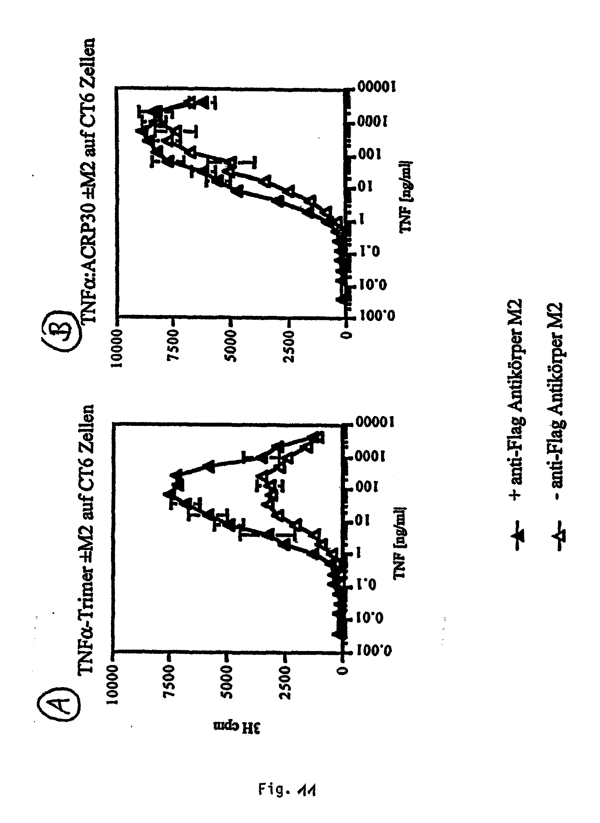

In Figur 11 wird das Ergebnis eines Zellproliferationsexperiments dargestellt. Hierbei wurde als Maß für die Zellproliferation von CT6-Zellen die Inkorporation von ³[H]-Thymidin in die CT6-Zellen von Mäusen als Funktion der Konzentration von Trimeren eines Fusionsproteins nach dem Stand der Technik (Flag- und Linker-Sequenz am N-Terminus mit nachfolgenden Aminosäuren 77 bis 235 aus murinem TNFα) ohne eine Komponente B, also des mTNFα-Trimers (Figur 11A), oder von erfindungsgemäßen Dodekameren (4x3) des erfindungsgemäßen Fusionsproteins (6) gemäß Fig. 10, des mTNFα-ACRF30 (Figur 11B), bestimmt. Die ³[H]-Thymidin-Inkorporation wird in "cpm" ("counts per minute") dargestellt. Die Versuche wurden in Gegenwart (▲) oder in Abwesenheit (Δ) von Anti-Flag-Antikörpern M2 vorgenommen. Dabei hat das aus dem Stand der Technik bekannte Trimer von mTNFα nach Bindung an den TNF-Rezeptor 2 (TNF-R2) nur einen geringen proliferativen Effekt auf die CT6-Zellen (Figur 11A, Δ). Erst nach Zugabe von Anti-Flag-Antikörpern (▲) durch deren vernetzende und damit oligomerisierende Wirkung kann ein deutlich erhöhter proliferativer Effekt beobachtet werden.

Demgegenüber zeigen erfindungsgemäße Oligomere der Trimere, hier das Dodekamer des erfindungsgemäßen Fusionsproteins (6), in Figur 11B eine starke proliferative Wirkung, die bereits bei 5 ng/ml beobachtet werden kann (Δ). Die Kombination von vernetzendem Anti-Flag-Antikörper und erfindungsgemäßen Dodekameren (▲) als Referenz kann demgegenüber nur noch eine geringfügige Erhöhung der proliferativen Wirkung im Vergleich zum Ergebnis bei alleiniger Zugabe des erfindungsgemäßen Dodekamers besorgen. Daraus ist zu erkennen, daß ein erfindungsgemäßes Oligomer, hier in Gestalt eines Dodekamers, bereits eine nahezu optimale Proliferationswirkung hervorruft.

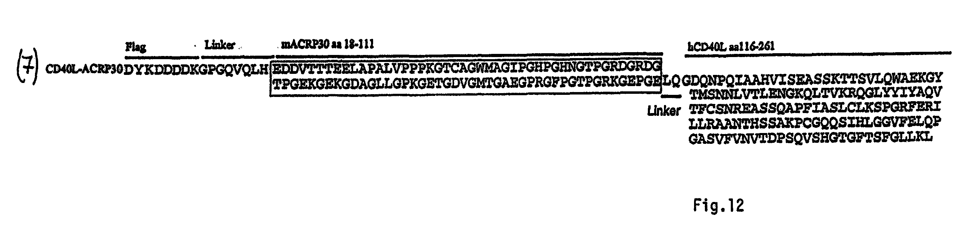

Die Aminosäuresequenz eines erfindungsgemäßen Fusionsproteins (7), das die Aminosäuren 116 bis 261 des hCD40L (h: human) als Komponente A in Kombination mit der Oligomerisierungsdomäne von ACRP30 (AS 18-111) als Komponente B enthält, ist in Figur 12 dargestellt (CD40L-ACRP30).

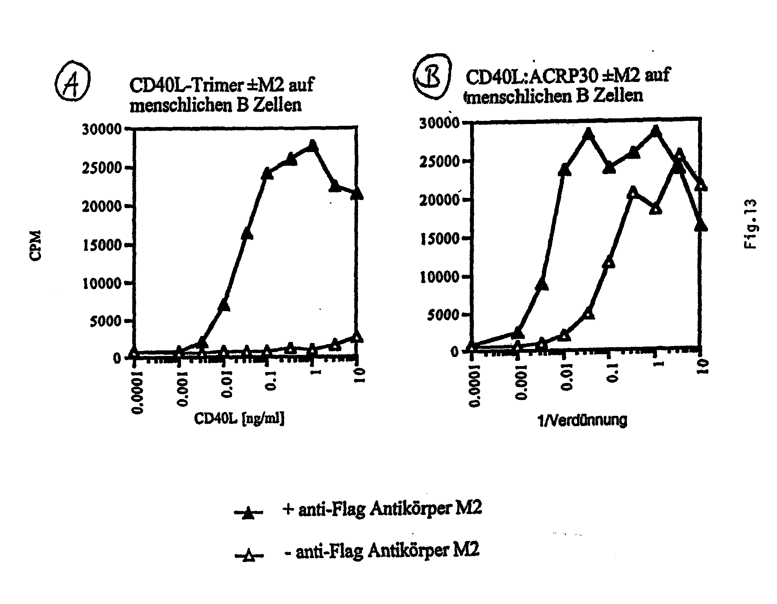

In Figur 13 wird - genauso wie in Figur 11 - das Ergebnis eines Zellproliferationsexperiments dargestellt. Hierbei wurden allerdings - im Unterschied zu Fig. 11 - humane PBL (periphere Blutlymphozyten) verwendet. Dargestellt ist die Wirkung von herkömmlichen CD40L, das in Lösung in Form eines Trimers (Flag- und Linker-Sequenz, wie in Figur 12, mit nachfolgender Sequenz von AS 16 bis 261 von hCD40L ohne eine oligomerisierende Komponente B; Figur 13A) vorliegt, auf die Proliferation der Zellen im Vergleich zur Wirkung von erfindungsgemäßen Dodekameren von erfindungsgemäßen Fusionsproteinen (7) gemäß Figur 12, CD40L-ACRP30 (Figur 13B). Die in Abwesenheit (Δ) von Anti-Flag-Antikörpern M2 vorgenommenen Versuche zeigen, daß das CD40L-Trimer nahezu keine proliferative Wirkung hat, während in Figur 13B bereits bei weitaus geringeren Konzentrationen an hCD40L eine entsprechende Proliferationswirkung nachweisbar ist. Auf der x-Achse findet sich bei Fig. 13B die Größe "1/Verdünnung", also nicht absolute Konzentrationen, aufgetragen, da ein angereicherter Überstand, dessen absolute Konzentration unbekannt ist, zugegeben wurde. Die Kombination von vernetzendem Anti-Flag-Antikörper und Dodekameren von erfindungsgemäßem Fusionsprotein (7) (▲) als Referenz kann durch Bildung von Aggregaten höherer Ordnung eine weitere Verschiebung einer entsprechenden Proliferationswirkung zu niedrigeren Konzentrationen erzeugen.

Figur 14 stellt schematisch die Struktur von oligomerisierten Multimeren dar. Die Figuren 14A und 14B zeigen schematisch das Aussehen von nativen "Bündelproteinen" mit ihrer bereits nativ oligomerisierten Struktur (Fig. 14A: Hexamer von Kopfdomänen-Trimeren, die das Komplementprotein C1q bilden; Fig. 14B: Tetramer von Kopfdomänen-Trimeren, die ACRP30 bilden, ein durch Adipozyten produziertes Serumprotein). Damit werden beim C1q- oder beim ACRP30-Komplex die Kopfdomänen der nativen Monomere multimerisiert und oligomerisiert. Während der C1q-Oligomerisierungskomplex aus drei verschiedenen Genprodukten aufgebaut ist, handelt es sich beim ACRP30-Oligomerisierungskomplex um ein Homododekamer, also um identische multi- und oligomerisierte Genprodukte. Die den beiden vorgenannten nativen Oligomerisierungskomplexen jeweils zugrundeliegenden einzelnen Polypeptidketten verfügen jeweils über eine Kopfdomäne, einen Sequenzbereich, der eine Kollagen-Tripelhelix bilden kann, und über einen Sequenzbereich, der 6 (C1q-Komplex) bzw. 4 (ACRP30-Komplex) Kollagen-Tripelhelices schließlich zu einem Helixbündel aus 18 bzw. 12 Monomeren oligomerisieren kann.

In Figur 14C wird schematisch ein erfindungsgemäßes oligomerisiertes Multimer eines erfindungsgemäßen Fusionsproteins (z.B. des Fusionsproteins (4) gemäß Fig. 6A, FasL-ACRP30) dargestellt, das als Komponente A vier trimerisierte TNF-Liganden (z.B. den TNF-Liganden FasL) oder Abschnitte von TNF-Liganden aufweist, die durch die Kollagen-artigen Sequenzbereiche von ACRP30-Protein als Komponente B, welche bspw. in einem erfindungsgemäßen Fusionsprotein vorliegen, zu einem erfindungsgemäßen Homododekamer oligomerisieren.

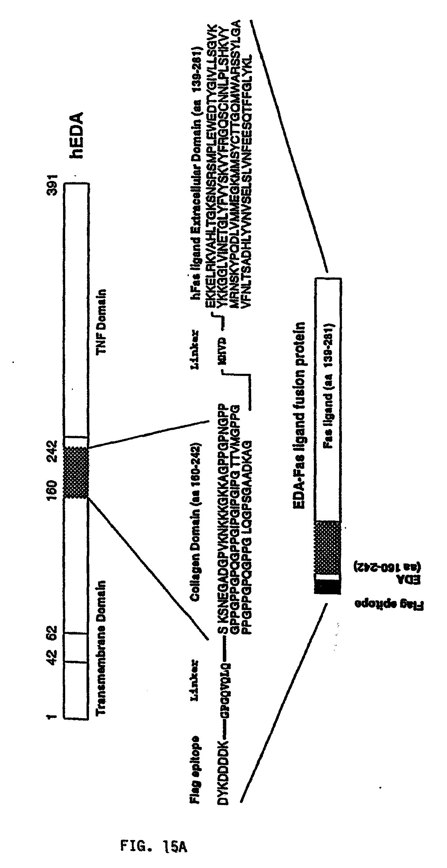

Fig. 15A beschreibt die Konstruktion eines weiteren erfindungsgemäßen Fusionsproteins, nämlich hEDA/FasL. Hierbei dient die Kollagendomäne von humanem EDA (Aminosäuren 160 bis 242) als Komponente B, an die N-Terminal über einen Linker ein Flag-Epitop und C-terminal, auch über einen Linker, eine Komponente A, in Fig. 15A FasL (AS 139-281, also die extrazelluläre Domäne von FasL oder ein Fragment derselben), angekoppelt ist. Das erfindungsgemäße Fusionsprotein hEDA/FasL liegt als Hexamer vor, 2x3mer. Beim Protein EDA handelt es sich um ein weiteres Mitglied der TNF-Familie, das neben einer Transmemebran-Domäne und einer TNF-Domäne auch eine Kollagen-Domäne aufweist (s. Fig. 15A oben) und in der humanen Form 391 AS umfaßt.

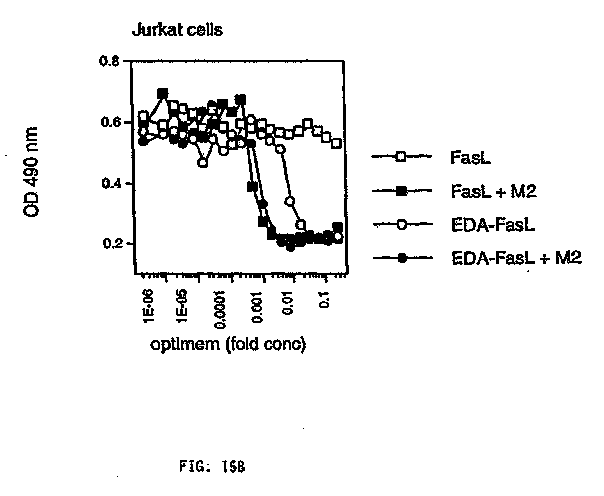

Figur 15B stellt Untersuchungen mit dem erfindungsgemäßen Fusionsprotein hEDA/FasL dar. Zur Herstellung des Fusionsproteins wurde zunächst die Kollagen-Domäne von humanem EDA (AS 160 bis 242) mit entsprechenden Primern amplifiziert und dann mit den entsprechenden Sequenzen am N- bzw. C-Terminus für Flag und die extrazelluläre Domäne von humanem FasL (AS 139 bis 281) fusioniert. Figur 15B stellt nunmehr Kurvenverläufe dar, die sich aus der Titration von Jurkat-T-Zellen mit einerseits FasL und andererseits dem Fusionsprotein hEDA/FasL unter Zugabe von Anti-Flag-Antikörper M2 und ohne Zugabe von Anti-Flag-Antikörper M2 ergeben. Hierzu wurden Überstände (OPTIMEM) von transient mit FasL oder hEDA/FasL transfizierten und diese Proteine exprimierenden 293-Zellen zu Jurkat-T-Zellen gegeben. In aufeinanderfolgenden Experimenten wurden die auf der x-Achse von Fig. 15B dargestellten, abnehmenden Konzentrationen der vorgenannten Proteine eingesetzt und die jeweilige Überlebensrate der Jurkat-Zellen mit Hilfe des an anderer Stelle beschriebenen Standard-Zytotoxizitätstests bestimmt. Aus der Auftragung wird deutlich, daß FasL ohne kreuzvernetzenden M2-Antikörper inaktiv ist (□) und in diesem Fall erst die kreuzvernetzende Wirkung des Antikörpers M2 zytotoxische Effekte hervorruft (■). Demgegenüber wird die entsprechende Wirkung von einem erfindungsgemäßen Fusionsprotein, nämlich hEDA/FasL, bereits ohne Zugabe von M2-Antikörper erreicht (o). Die Zugabe von M2-Antikörpern kann in diesem Fall die Wirkung des erfindungsgemäßen Fusionsproteins noch steigern (•).

### Die vorliegende Erfindung wird durch die nachfolgenden Ausführungsbeispiele näher erläutert:

Die folgenden experimentellen Gegebenheiten (a) bis (f) sind, soweit einschlägig und nicht entsprechende a.a.O. offenbarte Modifizierungen gelten, für die sechs nachfolgenden Ausführungsbeispiele heranzuziehen:

### (a) Vektorkonstruktionen für die FasL-, TRAIL-, TNFα- und CD40L-Fusionsproteine:

Ein für das Signalpeptid von Hämagglutinin codierendes DNA-Fragment, einschließlich 6 Basen der im 5'-Bereich unübersetzten Sequenz (CAA AAC ATG GCT ATC ATC TAC CTC ATC CTC CTG TTC ACC GCT GTG CGG GGC) sowie das Flag Epitop (GAT TAC AAA GAC GAT GAC GAT AAA), der Linker (GGA CCC GGA CAG GTG CAG), die Restriktionsschnittstellen *Pst*I*, Sal*I*, Xho*I und *Bam*HI wurden zwischen die Restriktionsschnittstellen *Hind*I-II und *Bam*HI eines modifizierten PCRIII-Vektors (InVitrogen, NV Leek, Niederlande) kloniert, in dem die Basen 720-769 deletiert wurden (PS 038). Für den Expressionsvektor des trimerischen FasL wurden die Aminosäuren 139 bis 281 der für menschlichen FasL kodierenden Sequenz, eingerahmt von den Restriktionsschnittstellen *Pst*I und *Eco*RI, mit PCR amplifiziert und in den modifizierten PCRIII-Vektor kloniert.

Für hexameren FasL wurde die für die Aminosäuren 103 bis 281 kodierende Sequenz, beidseitig eingerahmt von *Eco*RI Schnittstellen sowie zusätzlich am 5'-Ende von der Linkersequenz GGCTT und am 3'-Ende vom Stop-Codon (TAA) und der natürlichen, nicht übersetzten Sequenz (GAG AAG CAC TTT GGG ATT CTT TCC ATT ATG ATT CTT TGT TAC AGG CAC CGA GAT GTT GAA GCC) in die *Eco*RI-Schnittstelle des Vektors PS 038 kloniert. Der "Super-FasL" (Figur 4, Fusionsprotein (3)) wurde durch Einführung einer Punktmutation in der Linkersequenz des Vektors PS 038 erzeugt, indem die Sequenz CAGTGTGCTG auf der 5'-Seite der *Eco*RI-Schnittstelle durch CAGTCTGCAG mit Hilfe von PCR-Mutationsmethoden ersetzt worden ist. Daraufhin wurde der FasL (Aminosäuren 103 bis 281) in der zuvor beschriebenen Weise in den modifizierten PS 038 kloniert.

Für humanes TRAIL, murines TNFα und humanes CD40L wurden Teile der extrazellulären Domänen (TRAIL: Aminosäuren 95 bis 281, TNFα: Aminosäuren 77 bis 235, CD40L: Aminosäuren 116 bis 261) mit *Pst*I-Restriktionschnittstellen am 5'-Ende und einem Stop-Codon sowie *Spe*I- und *Eco*RI-Restriktionsschnittstellen am 3'-Ende durch PCR amplifiziert und in den Vektor PCRII (Invitrogen) kloniert. Für die Expression der Liganden als Trimere wurde zunächst die Sequenz GAT TAC AAA GAC GAT GAC GAT AAA, kodierend für den Flag-Tag, sowie die Linkersequenz GGA CCC GGA CAG GTG CAG zwischen die Schnittstellen *Bam*HI und PstI in den Vektor pQE-16 (Qiagen) inseriert (PS 330). Anschließend wurden die Liganden als *Pst*I/*Spe*I-Fragmente in PS 330 subkloniert.

Der Expressionsvektor für FasL-ACRP30 wurde in folgender Weise konstruiert. Mittels des EST-Klons AA673154 wurde durch PCR die für die Aminosäuren 18 bis 111 des murinen ACRP30 kodierende Sequenz, eingerahmt von den RestriktionsSchnittstellen *Nsi*I und *Pst*I in die *Pst*I-Schnittstelle des für trimeren FasL kodierenden Vektors kloniert (dergestalt, daß die fusionierte *Nsi*I/*Pst*I-Schnittstelle auf der 5'-Seite der kodierenden Sequenz lag). Die Vektoren zur Expression der Fusionsproteine der übrigen TNF-Cytokine mit ACRP30 wurden durch Substituierung der entsprechenden Sequenz von FasL im Expressionsvektor FasL-ACRP30 mit der jeweiligen Ligandensequenz in die Restriktionsschnittstellen *Pst*I und *Eco*RI erstellt.

### (b) Expression und Reinigung der rekombinanten Proteine:

Für die trimeren TRAIL, TNFα und CD40L wurden stabile Klone in Bakterien (Stamm M15 mit Plasmid pRep4, Qiagen) etabliert. Die jeweiligen Klone wurden über Nacht in LB-Broth mit Ampicillin (100 µg/ml) und Kanamycin (50 µg/ml) bei 37°C vorkultiviert und dienten zur Inokulation der Hauptkultur (Verdünnung 1:50, Wachstum bei 37°C), die nach einer Stunde für sechs Stunden mit 0,5 mM IPTG (Sigma) für die Expression induziert wurden. Die Bakterien wurden durch Zentrifugation geerntet, zweimal in PBS gewaschen, in der "French Press" lysiert, und das Lysat von den unlöslichen Abfällen durch Zentrifugation abgetrennt. Für alle FasL-Proteine wurden stabile Klone in HEK293-Zellen mittels Selektion in 800 µg/ml G418 erstellt (siehe auch a.a.O: Schneider et al., J. Exp. Med. 1998).

Die trimerischen und hexamerischen Liganden sowie Super-FasL wurden aus den Überständen stabiler Klone oder der bakteriellen Lysate mittels Affinitätschromatographie auf M2-Agarose (Sigma, Schweiz) aufgereinigt, mit 50 mM Citrat-NaOH (pH = 2,5) eluiert und alsbald mit 0,2 Volumina Tris-HCl (pH = 8) neutralisiert. Der Puffer wurde gegen PBS in Konzentratoren ausgetauscht (Centrikon-30, Amicon Corp., Easton, TX, USA).

Fusionen von FasL mit murinem ACRP30 wurden in der folgenden Weise aufgereinigt. Die Überstände wurden mit 50 mM NaCl und 1 mM CaCl₂ versetzt und der pH-Wert mittels Salzsäure/Natronlauge auf 7,0 eingestelt. Daraufhin wurde das rekombinante Protein auf M1-Agarose (Sigma, Schweiz) gebunden und in TBS-EDTA (10 mM) eluiert. Der Puffer wurde gegen PBS in Konzentratoren ausgetauscht. Die Konzentration von gereinigten Proteinen wurde durch das Bicinchoninsäure-Verfahren (Pierce Chemical Co., Rockford, IL, USA) unter Verwendung von Rinderserumalbumin als Standard bestimmt und die Reinheit der Proben durch SDS-PAGE und Coomassie-Blue-Färbung ermittelt.

Die Fusionsproteine von TRAIL, TNFα und CD40L mit ACRP30 wurden in den jeweiligen Assays in Form angereicherter Überstände hinzugefügt, die in folgender Weise hergestellt wurden: 293T-Zellen wurden mit den jeweiligen Expressionsplasmiden durch die Calciumphosphat-Methode transfiziert. Nachdem die Zellen 16 Stunden mit dem Präzipitat inkubiert worden waren, wurden die Zellen mit PBS gewaschen und 4 Tage in serumfreiem Medium (Optimem, Gibco BRL) inkubiert. Die Überstände wurden auf ein Zwanzigstel ihrer anfänglichen Volumina durch Konzentratoren reduziert und die Präsenz des Proteins durch "Western-Blotting" überprüft. Im Falle von TRAIL-ACRP30 und TNFα-ACRP30 wurden Titrationen dieser Proteine mit Titrationen von aufgereinigtem trimerischem TRAIL oder TNFα verglichen, um die jeweilige Konzentration der rekombinanten Proteine zu bestimmen.

### (c) Überprüfung des Molekulargewichts der Multimere durch Gelpermeationschromatographie:

Die Größe der verschiedenen Fusionsproteine wurde durch Gelfiltration auf Superdex 200 HR10/30 (Pharmacia) bestimmt. Rekombinantes Protein wurde mit den internen Standards Katalase und Ovalbumin im Falle der tri- und hexamerischen Liganden, mit Ferritin und Ovalbumin für die ACRP30-Fusionsproteine auf die Kolonne in einem Volumen von 200 µl geladen, dann mit PBS (0,5 ml/min) eluiert und in Fraktionen von 0,25 ml gesammelt. Die Präsenz der Proteine in den verschiedenen Fraktionen nach Fällung durch Trichloroessigsäure wurde durch "Western-Blotting" bestimmt. Die Größe der Proteine wurde mit Hilfe einer Regressionsgeraden, bestehend aus Thyroglobulin (669 kDa), Ferritin (440 kDa), Katalase (262 kDa), Aldolase (158 kDa), Rinderserumalbumin (67 kDa), Ovalbumin(43 kDa), Chymotrypsinogen A (25 kDa) und Ribonuclease A (13,7 kDa) ermittelt.

### (d) Zellen:

Murine B-Lymphom A20-Zellen wurden in DMEM gehalten, das 5% hitzeinaktiviertes FCS enthielt. Die humanen T-Lymphoplastom-Jurkat-Zellen, BJAB Burkitt-Lymphomzellen wurden in RPMI, begleitet von 10% FCS, aufgezogen. Die human-embryonischen Nierenzellen 293 wurden in einem DMEM Mehrstoffmix F12 (1:1), begleitet von 2% FCS, kultiviert. Alle Medien enthielten Antibiotika (Penicillin und Streptomycin bei 5µg/ml jeweils und Neomycin bei 10 µg/ml). Die IL-2 abhängige murine zytotoxische T-Zellinie CT6, wurde im RPMI, unterstützt von 10% FCS, 1 mM Natriumpyruvat, 50 µM 2 Mercaptoethanol, 10 mM Hepes und 10% konditioniertem EL-4 Zellüberstand aufgezogen.

### (e) Zytotoxischer Assay:

Der zytotoxische Assay wurde im wesentlichen, wie zuvor bei Schneider et al. (J. Biol. Chem. 272:18827-18833, 1997) beschrieben durchgeführt. Hierbei wurden 50.000 Zellen für die Dauer von 16 Stunden in 100 µl Medium inkubiert, wobei das Medium die angezeigten Ligandenkonzentrationen in Gegenwart oder Abwesenheit von 1µg/ml M2-Antikörper (2 µg/ml für TRAIL) enthielt. Die Zellüberlebensraten wurden mit Hilfe von PMS/MTS (Phenanzinmethosulfat 3-[4,5-dimethylthiazol-2-yl]-5-[3-carboxymethoxyphenyl]-2-[4-sulfophenyl]-2H-tetrazolium, Salz) (Promega Corp., Madison, WI) bestimmt. Die Farbentwicklung wurde für die erforderliche zeit (typischerweise 1-3 Stunden) zugelassen. Die Absorbanz wurde bei 490 nm gemessen.

### (f) B-Zell-Proliferationsassay

CD19-positive Zellen wurden durch FACS-Sorting von menschlichen PBL (periphere Blutlymphozyten) mit magnetischen "Beads" selektiert und aufgereinigt, dagegen wurden CD19-negative Zellen mit 3000 rad irradiert. 100000 gereinigte CD19-positive Zellen wurden mit 100000 CD19-negativen autologen irradierten PBL in 120 µl Medium (RPMI 10% FCS, Antibiotika) mit den titrierten löslichen CD40L-Fusionsproteinen, mit oder ohne M2- Antikörper (10 µg/ml) 72 Stunden lang in 96-"well"-Platten inkubiert. Anschliessend wurden die Zellen 6 Stunden lang mit [³H]-Thymidin gepulst und die Inkorporation wurde mit Flüssig-Szintillationszählung ("Liquid Scintillation Counting") gemessen.

Im übrigen wird hinsichtlich der Beschreibung der für die Durchführung der Ausführungsbeispiele eingesetzten Methoden ausdrücklich auf Schneider et al. (J. Exp. Med., Vol. 187, Nr. 8, 1998, 1205-1213) und die dort als Referenzen zitierten Publikationen verwiesen.

### 1. Ausführungsbeispiel

Es wurde ein rekombinantes Fusionsprotein (2) exprimiert, das die Aminosäuren 103 bis 281 des hFasL (h: human) als Komponente A und N-terminal von der Aminosäure 103 der Komponente A als Komponente B eine 18 AA lange Sequenz (VDLEGSTSNGRQCAGIRL, sog. spezifischer Linker) aufwies. Darüber hinaus wurde am N-Terminus des Fusionsproteins (N-Terminal von der Komponente B) eine Flag-Sequenz mit den Aminosäuren DYKDDDDK und einer darauf folgenden Linkersequenz GPGQVQLQ angekoppelt (Figur 1).

Für Vergleichsversuche wurde ein Fusionsprotein (1) exprimiert, das N-terminal gleichfalls die vorgenannte Flag-Sequenz mit der gleichen C-terminal folgenden Linkersequenz und daran C-terminal angeschlossen die Aminosäuren 139 bis 281 von hFasL aufwies. Fusionsprotein (1) unterschied sich von Fusionsprotein (2) demnach durch eine Deletion, die den spezifischen Linker und die Aminosäuren 103 bis 138 von hFasL umfaßte (Figur 1).

Die Vektorkonstruktion der Fusionsproteine (1) und (2) erfolgte nach der gemäß (a) beschriebenen Verfahrensweise. Die Expression und Reinigung der Fusionsproteine erfolgte nach dem unter (b) dargestellten Verfahren.

Die gereinigten Fusionsproteine (1) wurden reduzierenden oder nicht-reduzierenden Bedingungen unterworfen und anschließend gelelektrophoretisch aufgetrennt (Figur 2), verbunden mit einer Grobbestimmung des Molekulargewichts der jeweiligen Banden.

Schließlich wurden gemäß (d) gezüchtete A20- und Jurkat-Zellen entnommen und einem zytotoxischen Assay gemäß (e) unterzogen. Für jede der beiden Zellinien wurde der Assay jeweils mit steigenden Konzentrationen von trimerisiertem Fusionsprotein (1) oder von Bimeren von Trimeren (also Hexameren) des Fusionsproteins (2) in Gegenwart oder Abwesenheit von anti-Flag-M2-Antikörpern (Sigma, Buchs, Schweiz) durchgeführt (Figur 3), indem die Absorbanz bei OD 490 nm bestimmt wurde.

### 2. Ausführungsbeispiel

Es wurde ein rekombinantes Fusionsprotein (3) exprimiert, das die Aminosäuren 103 bis 281 des hFasL als Komponente A und N-terminal von der Aminosäure 103 der Komponente A als Komponente B eine 18 AA lange Sequenz (VDLEGSTSNGRQSAGIRL, spezifischer Linker) aufwies. Darüber hinaus wurde am N-Terminus des Fusionsproteins (N-Terminal von der Komponente B) eine Flag-Sequenz mit den Aminosäuren DYKDDDDK und einer darauf folgenden Linkersequenz GPGQVQLQ angekoppelt (Figur 4).

Für Vergleichsversuche wurde wie beim 1. Ausführungsbeispiel das Fusionsprotein (1) exprimiert (Figur 4).

Die Vektorkonstruktionen der Fusionsproteine (3) und (1) wurden nach den für das 1. Ausführungsbeispiel dargestellten Verfahren durchgeführt. Die Expression und Reinigung der Fusionsproteine erfolgte nach dem unter (b) dargestellten Verfahren.

Schließlich wurden gemäß (d) gezüchtete A20- und Jurkat-Zellen entnommen und einem zytotoxischen Assay gemäß (e) unterzogen. Für jede der beiden Zellinie wurde der Assay jeweils mit steigenden Konzentrationen von Trimeren des Fusionsproteins (3) in Gegenwart oder Abwesenheit von anti-Flag-M2-Antikörpern (Sigma, Buchs, Schweiz) durchgeführt (Figur 5), indem die Absorbanz bei OD 490 nm bestimmt wurde.

### 3. Ausführungsbeispiel

Es wurde ein rekombinantes Fusionsprotein (4) exprimiert, das die Aminosäuren 139 bis 281 des hFasL als Komponente A und N-terminal von der Aminosäure 139 der Komponente A zunächst das Linker-Dimer mit der Sequenz LQ und dann weiter N-terminal als Komponente B eine 94 AS lange Sequenz aus dem Protein mACRP30 (AS 18 bis 111), die Oligomerisierungsdomäne, aufwies. Darüber hinaus wurde am N-Terminus des Fusionsproteins (4) (N-Terminal von der Komponente B) eine Flag-Sequenz mit den Aminosäuren DYKDDDDK und einer darauf folgenden Linkersequenz GPGQVQLH angekoppelt (Figur 6A). Für Vergleichsversuche wurde das Fusionsprotein (1) eingesetzt.

Die Expression und Reinigung der Fusionsproteine erfolgte nach dem unter (b) dargestellten Verfahren.

Schließlich wurden gemäß (d) gezüchtete BJAB-Burkitt-Lymphoma-Zellen entnommen und einem zytotoxischen Assay gemäß (e) unterzogen. Der Assay wurde jeweils mit steigenden Konzentrationen von Trimeren des Fusionsproteins (1) oder oligomerisierten Trimeren (Dodekamere) des Fusionsproteins (4), also des rekombinaten FasL-ACRP30 (4x3), in Gegenwart oder Abwesenheit von anti-Flag-M2-Antikörpern (Sigma, s.o.) durchgeführt (Figur 7), indem die Absorbanz bei OD 490 nm bestimmt wurde.

### 4. Ausführungsbeispiel

Es wurde ein rekombinantes Fusionsprotein (5) exprimiert, das die Aminosäuren 95 bis 281 des hTRAIL (h: human) als Komponente A und N-terminal von der Aminosäure 95 der Komponente A zunächst der Linker-Dimer mit der Sequenz LQ und dann weiter N-terminal als Komponente B eine 94 AS lange Sequenz aus dem Protein mACRP30 (AS 18 bis 111), die Oligomerisierungsdomäne) aufwies. Darüber hinaus wurde am N-Terminus des Fusionsproteins (N-Terminal von der Komponente B) eine Flag-Sequenz mit den Aminosäuren DYKDDDDK und einer darauf folgenden Linkersequenz GPGQVQLH angekoppelt (Figur 8).
Für Vergleichsversuche wurde ein Fusionsprotein (nicht in der Figur 8 dargestellt) exprimiert, das in Lösung als Trimer vorliegt (TRAIL-Trimer). Dieses Vergleichsversuchsprotein weist (vom N- zum C-Terminus die Flag-Sequenz, den Linker mit der Sequenz GPGQVQLH und schließlich hTRAIL (AS 95 bis 281) auf. Im Unterschied zum Fusionsprotein (5) fehlt also die Komponente B (mACRP30: AS 18 bis 111) und der Linker mit der Sequenz LQ.

Die Expression und Reinigung der Fusionsproteine erfolgten nach den unter (b) dargestellten Verfahren.

Schließlich wurden gemäß (d) gezüchtete T-Lymphoblastoma-Jurkat-Zellen entnommen und einem zytotoxischen Assay gemäß (e) unterzogen. Der Assay wurde jeweils mit steigenden Konzentrationen von Trimeren des Fusionsproteins ohne die ACRP30-Sequenz (zum Vergleich) oder der oligomerisierten Trimeren des Fusionsproteins (5), also eines Dodekamers des rekombinaten TRAIL-ACRP30 (4x3), in Gegenwart oder Abwesenheit von anti-Flag-M2-Antikörpern (Sigma, s.o.) durchgeführt (Figur 9), indem die Absorbanz bei OD 490 nm bestimmt wurde.

### 5. Ausführungsbeispiel

Es wurde ein rekombinantes Fusionsprotein (6) exprimiert, das die Aminosäuren 77 bis 235 des mTNFα (m: murin) als Komponente A und N-terminal von der Aminosäure 85 der Komponente A zunächst das Linker-Dimer LQ und dann weiter N-terminal als Komponente B eine 94 AS lange Sequenz aus dem Protein mACRP30 (AS 18 bis 111), die Oligomerisierungsdomäne, aufwies. Darüber hinaus wurde am N-Terminus des Fusionsproteins (N-Terminal von der Komponente B) eine Flag-Sequenz mit den Aminosäuren DYKDDDDK und einer darauf folgenden Linkersequenz GPGQVQLH angekoppelt (Figur 10).

Für Vergleichsversuche wurde ein Fusionsprotein (nicht in der Figur 10 dargestellt) exprimiert, das in Lösung als Trimer vorliegt (TNFα-Trimer). Dieses Vergleichsversuchsprotein weist (vom N- zum C-Terminus die Flag-Sequenz, den Linker mit der Sequenz GPGQVQLH und schließlich mTNFα (AS 77 bis 235) auf. Im Unterschied zum Fusionsprotein (6) fehlt also die Komponente B (mACRP30: AS 18 bis 111) und der Linker mit der Sequenz LQ.

Die Expression und Reinigung der Fusionsproteine erfolgten nach dem unter (b) dargestellten Verfahren.

Mit Hilfe eines Zellproliferationsassays gemäß (f) wurden die Effekte, die durch Zugabe von steigenden Konzentrationen von TNFα-Trimeren oder TNFα-ACRP30-Oligomeren (Homododekamer von TNFα) in Gegenwart oder Abwesenheit von anti-Flag-M2-Antikörpern (Sigma, s.o.) zu CT6-Zellen ergeben, gemessen.
Hierzu wurden CT6-Zellen für die Dauer von 4 Tagen in Gegenwart von reduzierten Konzentrationen von EL-4-Überstand (2,5%) vor dem Proliferationsexperiment angesetzt. Die Zellen wurden in 96-"well"-Titerplatten (40.000 Zellen/"well") für die Dauer von 16 Stunden mit den angezeigten Konzentrationen von TNFα-ACRP30-Oligomeren oder mTNF-α in Gegenwart oder in Abwesenheit von 2 µg/ml M2-mAk und in der Abwesenheit von EL-4-Überstand inkubiert. Die Zellen wurden für die Dauer von zusätzlichen 6 Stunden mit [³H]Thymidin (0,5 µCi/"well") gepulst, drei Zyklen von Einfrieren und Auftauen ausgesetzt und schließlich geerntet. Die [³H]Thymidin-Inkorporation wurde schließlich durch eine Flüssigszintillationsmessung überprüft (Figur 11).

### 6. Ausführungsbeispiel

Es wurde ein rekombinantes Fusionsprotein (7) exprimiert, das die Aminosäuren 116 bis 261 des hCD40L (h: human) als Komponente A und N-terminal von der Aminosäure 95 der Komponente A zunächst das Linker-Dimer LQ und dann weiter N-terminal als Komponente B eine 94 AS lange Sequenz aus dem Protein mACRP30 (AS 18 bis 111), die Oligomerisierungsdomäne), aufwies. Darüber hinaus wurde am N-Terminus des Fusionsproteins (N-Terminal von der Komponente B) eine Flag-Sequenz mit den Aminosäuren DYKDDDDK und einer darauf folgenden Linkersequenz GPGQVQLH angekoppelt (Figur 12).

Für Vergleichsversuche wurde ein Fusionsprotein (nicht in der Figur 12 dargestellt) exprimiert, das in Lösung als Trimer vorliegt (CD40L-Trimer). Dieses Vergleichsversuchsprotein weist (vom N- zum C-Terminus die Flag-Sequenz, den Linker mit der Sequenz GPGQVQLH und schließlich hCD40L (AS 116 bis 261) auf. Im Unterschied zum Fusionsprotein (7) fehlt also die Komponente B (mACRP30: AS 18 bis 111) und der Linker mit der Sequenz LQ.

Die Expression und Reinigung der Fusionsproteine erfolgten nach den unter (b) dargestellten Verfahren.

Schließlich wurde der unter (f) beschriebene Zellproliferationsassay analog auf PBL durchgeführt, wobei CD40L-Trimer oder CD40L-ACRP30-Oligomere (Homododekamere) in An- oder Abwesenheit von anti-Flag-M2-Antikörpern (Sigma, s.o.) zugegeben wurden (Figur 13).

### 7. Ausführungsbeispiel

### 7.1 Experimentelle Durchführung

Das 7. Ausführungsbeispiel befaßt sich mit Fusionsproteinen, die aus einer multi- und oligomerisierenden Komponente A und einem Rezeptor als Komponente B bestehen.

### (A) Vektorkonstruktion

Die Fusionsproteine wurden aus einem modifizierten PCR-3-Vektor (von Invitrogen) als Folge von austauschbaren Modulen in der folgenden Reihenfolge (5' nach 3') konstruiert: (a) ein HindIII/SalI-Modul, das die extrazelluläre Domäne eines Rezeptors enthält, wobei eine Kozak-Sequenz GCCACC vorangeht (im Falle von hTNF-R1 (Aminosäuren 1 - 211); h-TRAIL-R1 (Aminosäuren 1 - 239), h-TRAIL-R2 (Aminosäuren 1 - 212), h-TRAIL-R3 (Aminosäuren 1 - 240) und hCD40 (Aminosäuren 1 -193) oder im Falle von hFasR (Aminosäuren 1 - 170) durch die Voranstellung von 24 Nukleotiden der 5'-nichttranslatierten Region); (b) ein 14-Aminosäuren langer Linker (PQPQPKPQPKPEPE) in einer SalI/XhoI-Kassette (wie bei Terskikh et al. (1997), Proc. Natl. Acad. Sci. USA 94, 1663 beschrieben); (c) eine Oligomerisierungsdomäne in einem Xhol/NotI-Modul im Falle von OPG (Aminosäuren 187 - 401, hier als δN-OPG beschrieben), CMP (Aminosäuren 451 - 493), GenBank 115555); COMP (Aminosäuren 32 - 75, GenBank 1705995); (d) eine NotI/XbaI-Kassette, die eine kombinierte His₆-myc-Markierung und ein Stop-Kodon enthält. Die Oligomerisierungsdomäne wurde umrahmt von den Aminosäuresequenzen GGCC und ARTPGGGS an den N- bzw. C-Termini. Linker wurden für alle Konstrukte eingesetzt. Im Falle der Fc-Konstrukte wurden die "Hinge"-Region, die CH2 und CH3-Domänen und das Stop-Kodon von hIgG1 als SalI/NotI-Kassette, wie zuvor beschrieben (Schneider et al. (1997) J. Biol. Chem 272, 18827; Schneider et al. (1998) J. Exp. Med. 187, 1205), kloniert. Stabile HEK-293 abgeleitete Zellinien wurden für die Herstellung von rekombinanten Proteinen durch Selektion in 800 µg/ml G418 mit Hilfe der zuvor beschriebenen Verfahren (Schneider et al. (1997) s.o.), etabliert.

### (B) Transiente Transfektion

Die 293T-Zellen wurden mit der CaCl₂-Methode wie zuvor beschrieben (Schneider et al. 1997, s.o.) transfiziert und mit PBS gewaschen, bevor sie für 3 Tage in serumfreien Optimem-Medium inkubiert wurden. Die Überstände wurden 30fach konzentriert und bis zum Gebrauch gefroren gehalten. Die Konzentration der Fas/δN-OPG und Fas/CMP-Fusionsproteine in konzentriertem Optimem-Medium wurde mit Hilfe einer Titration auf "Western Blots" unter Zuhilfenahme von aufgereinigtem Fas/COMP als Standard bestimmt.

### (C) Reinigung der rekombinanten Proteine

Die Überstände von stabil transfizierten 293-Zellen wurden auf M2-Agarose (als Liganden) oder Protein A-Sepharose (Fc-Fusionsproteine) geladen, mit PBS gewaschen und mit 50 mM Citrat-NaOH (pH 2,5) eluiert. Das Eluat wurde mit Tris-HCl (pH 8) neutralisiert und der Puffer gegen PBS in Centrikon-30-Konzentratoren (von Amicon, Easton, Texas) ausgetauscht. Die COMP- und CMP-Fusionsproteine wurden auf HiTrap-Chelatsäulen gereinigt. Zu diesem Zweck wurden die Überstände von stabil transfizierten 293-Zellen mit 500 mM NaCl und 10 mM Imidazol ergänzt und über mit 0,5 M ZnSO₄ (pH 2,5) vorbeschichtete Säulen geführt und in PBS äquilibriert. Die Säule wurde mit PBS gewaschen, und die Proteine wurden mit PBS, das 50 mM EDTA enthielt, eluiert. Der Puffer wurde gegen PBS ausgetauscht, wie zuvor beschrieben.

Flag-FasL und Flag-TRAIL wurdenso hergestellt, wie zuvor beschrieben (Schneider et al., 1997, J. Biol. Chem. 272, 18827 und Thome et al., 1997, Nature 386, 517). Beide Veröffentlichungen sind vollumfänglich Bestandteil der Offenbarung des vorliegenden Erfindungsgegenstands. Flag-CD40L (AS 116 bis 261) wurde in Bakterien exprimiert und auf M2-Agarose gereinigt, wie es für Flag-TRAIL beschrieben wurde. Die Proteinkonzentrationen wurden mit der Bicinchoninsäure-Methode bestimmt. Der Reinheitsgrad wurde mit SDS-PAGE und Coomassie-Blue-Markierung untersucht.

### (D) Gelpermeationschromatographie

Im Rahmen der Gelpermeationschromatographie wurde die jeweilige Menge von Fusionsprotein in einem Volumen von 200µl auf eine Superdex 200 HR 10/30-Säule (von Pharmacia) geladen und mit 0,5 ml/min in PBS eluiert, wobei gleichzeitig die Absorbanz bei 280 nm aufgezeichnet wurde. Die einzelnen Fraktionen (0,25 ml) wurden - wie nachfolgend beschrieben - in Zytotoxitätstests analysiert. Zur Bestimmung des Molekulargewichts wurde die Säule mit Standardproteinen Thyroglobulin (669 kD), Ferritin (440 kD), Katalase (262 kD), Aldolase (158 kD), Rinderserumalbumin (67 kD), Hühnchenovalbumin (43 kD), Chymotrypsinogen A (25 kD) und Ribonuklease A (13,7 kD) kalibriert.

### (E) KompetitionsELISA

Der KompetitionsELISA wurde, wie nachfolgend beschrieben, durchgeführt. 96 "Well"-ELISA-Platten wurden mit Rezeptor/Fc-Fusionsprotein (0,2 µg/ml in PBS, 100µl, 16 Stunden, 25°C) beschichtet. Die "Wells" wurden für eine Stunde bei 37°C in PBS gesättigt, wobei das PBS 5% fötales Kälberserum (als blockierenden Puffer) enthielt. Bei Einsatz von sCD40L war der blockierende Puffer PBS, enthaltend 4% fettfreie Milch und 0,05% Tween-20. Kompetitierende Fc oder COMP-Fusionsproteine wurden seriell in 100 µl blockierendem Puffer in Gegenwart oder Abwesenheit von 1 µg/ml Protein A gelöst. Die Liganden wurden bei konstanter Konzentration hinzugefügt (sFasL: 2 µg/ml, 36 nM, sTNFα: 0,02 µg/ml, 0,36 mM; sTRAIL: 0,1 µg/ml, 1,4 nM; sCD40L: 0,5 µg/ml, 9,2 nM, alles in blockierendem Puffer) und konnten für die Dauer von 1 Stunde bei 37°C binden. Gebundene Liganden wurden mit M2-anti-Flag-Antikörper (1 µg/ml in blockierendem Puffer, 100 µl, 45 Minuten, 37°C), Ziegen-Antimaus-Antikörper, gekoppelt an Peroxidase, 1:2000 in blockierendem Puffer, 100 µl, 30 Minuten, 37°C) und o-Phenylendiaminhydrochlorid (0,3 mg/ml in 50 mM Zitronensäure, 100 mM Na₂HPO₄, 0,01% H₂O₂) identifiziert. Die Absorbanz wurde bei 490 nm gemessen.

### (F) Zytotoxizitätstests

Die Zytotoxitätstests wurden in 96 "Well"-Platten in einem Volumen von 100 µl im wesentlichen wie bei Schneider et al. (1997), s.o., beschrieben, durchgeführt. Die chimärischen Rezeptoren wurden in einem Medium, das solche Mengen von cytotoxischen Liganden enthielt, die mehr als 95% Zelltod induzieren konnten, seriell verdünnt. Wo es angegeben ist, wurde Protein A mit einer Konzentration von 1 µg/ml eingesetzt. sFasL wurde in Gegenwart von 1 µg/ml M2 eingesetzt und sTRAIL in Gegenwart von 2 µg/ml M2. Kein M2-Antikörper wurde bei den Testreihen mit sTNFα verwendet. Die zellen wurden einer 16stündigen Inkubation ausgesetzt und ihre Überlebensraten unter Gebrauch des PMS/MTS-Testsystems (Phenazinmethosulfat/3-[4,5-dimethylthiazol-2-yl]-5-[3-carboxymethoxyphenyl]-2-[4-sulfophenyl]-2H-tetrazolium, in Salzform) (Promega, Madison, Wisconsin). Die Absorbanz wurde bei 490 nm gemessen. Um die molaren Konzentrationen der verschiedenen Fusionsproteine angeben zu können, wurde das Molekulargewicht, wie folgt, geschätzt: [(theoretisches Mᵣ+3 kDa pro vorhergesagtem N-gekoppeltem Glyan)x Multiplizität]. Fas:Fc, :COMP, :CMP, :δN-OPG: 98, 172, 100 bzw. 105 kDa. TRAILR2:Fc, :COMP, :CMP, :δN-OPG: 86, 142, 82 und 93 kDa. TNFR1:Fc, :COMP: 104 und 187 kDa. CD40:Fc, :COMP: 101 and 180 kDa. TRAILR2:Fc: 86kDA, TRAILR3:Fc: 127 kDA. Flag-TRAIL, Flag-FasL, Flag-TNFα, Flag-CD40L: 71, 55, 56 bzw. 54 kDa.

### (G) BIAcore-Messungen

Die BIAcore-Messungen wurden auf CM5-Carboxymethyldextran modifizierten Sensorchips (von BIAcore AB, Uppsala, Schweden) bei einer Durchflußgeschwindigkeit von 5µl/min durchgeführt. Die CM5-Chips wurden mit einer 50µl-Gabe einer 1:1 Mischung von N-Ethyl-N'(3-dimethylaminopropyl)-carbodiimid:N-hydroxysuccinimid aktiviert. Sechs µl einer 100µg/ml Lösung von M2-anti-Flag monoklonalem Antikörper in 10 mM NaHCO₃ (pH 5,5) wurden dann über die aktivierte Oberfläche geführt. Eine 50µl-Gabe von 1M Ethanolamin-HCl deaktivierte die verbleibenden N-Hydroxysuccinimidester. Die Menge von immobilisiertem M2-Antikörper, die für diese Vorgehensweise gebraucht wurden, lag bei ungefähr 4600 Einheiten. Zur Analyse der Interaktionen von FasL-Fas:Fusionsprotein und TRAIL-TRAIL-R2:Fusionsprotein wurde eine konstante Menge von markiertem Ligand auf einer M2 modifizierten Oberfläche immobilisiert. Um dies zu erreichen, wurde ein 7µl-Gabe von 2,5 µg/ml Flag-FasL oder Flag-TRAIL auf die Oberfläche aufgebracht. Dies führte zur Bindung von ungefähr 100 bis 150 Einheiten. Diese Bedingungen gestatteten im übrigen eine minimale Dissoziation der Liganden von der M2-Oberfläche, während sie gleichzeitig eine ausreichende nachfolgende Rezeptorbindung für die Analyse gestatteten. Das Binden des Rezeptor:Fusionsproteins wurde dann durch 15µl-Injektion von gereinigtem Rezeptor:Fusionsprotein bei Konzentrationen zwischen 1 und 100 µg/ml, entsprechend 100 bis 150 Einheiten, analysiert. Die Assoziierungskinetik wurde für die Dauer von 3 Minuten gemessen und die Dissoziationsgenetik für die Dauer von 3 bis 5 Minuten. Die Oberfläche wurde dann regeneriert (zu einer einfachen M2-Oberfläche) durch eine 5µl-Gabe von 50 mM Zitrat-HCl (pH 2,5). Bis zu 30 aufeinanderfolgende Abläufe von Bindung und Regeneration konnten ohne signifikante Veränderung der Bindungsgeigenschaften von immobilisiertem M2-Antikörper durchgeführt werden. Die Dissoziations- und Assoziationskinetiken wurden unter Zuhilfenahme des Kinetik-Analyseprogramms des Herstellers unter Verwendung der Modelle AB=A+B und A+B=AB analysiert.

### (H) Kultivierung von primären Mäuse-Hepatozyten

Für die Kultivierung von primären Maus-Hepatozyten wurden C57BL/6 Mäuse geopfert, und der Leberbereich oberhalb des Biliargefäßes sofort entfernt, um ihn dann in einem "Hepatozyten-Attachment Medium" (HAM) zu halten. Der Leberbereich wurde zunächst mit 16 ml von 10mM Hepes (pH 7,6) perfundiert, um die Erythrocyten zu beseitigen, dann mit 12 ml von 0,5 mg/ml Collagenase H (von Boehringer Mannheim)in Hepes (4 mM CaCl₂) behandelt und dann in einer Petrischale in Hepes homogenisiert. Die Zellen wurden in Hepes gewaschen, dann zentrifugiert (100xg, 30 Sekunden) und in 60%iger isotonischer Percoll-Lösung (von Pharmacia) in HAM resuspendiert und wiederum zentrifugiert (700xg, 2 min). Alle Puffer und das Medium wurden bei 37°C eingesetzt. Die sedimentierten Zellen wurden in HAM resuspendiert, gezählt, auf flachschaligen Mikrotiterplatten ausgesetzt (10 000 pro Well, 200 µl) und konnten entsprechend anhaften. Die experimentelle Mischung (seriell verdünnte Inhibitoren, sFasL (Endkonzentration: 400 ng/ml, 7 nM) und M2-Antikörper (Endkonzentration: 1 µg/ml)) wurden hinzugefügt, und die Zellen dann für weitere 16 Stunden inkubiert. Der Überstand wurde abgenommen, frisches HAM (100 µl) hinzugefügt, und der Überlebensfähigkeitstest PMS/MTS, wie oben beschrieben, durchgeführt.

### (I) Aktivierungsinduzierter Zelltod

Flachschalige Mikrotiterplatten wurden mit anti-humanem CD3 TR66 (10 µg/ml) in PBS für die Dauer von 3 Stunden bei 37°C beschichtet. Die Platten wurden zweimal in PBS gewaschen und einmal mit RPMI 1640 Medium. Jurkat-Zellen (5 x 10⁵ Zellen pro ml, 100 µl) wurden mit den Inhibitoren gemischt und auf jedes "Well" verteilt, dann zentrifugiert (200xg, 3 min) und für die Dauer von 24 Stunden bei 37°C inkubiert. Die Zellenüberlebensfähigkeit (bei OD 490 nm) wurde, wie oben beschrieben, gemessen. Die spezifische Zellprotektion (in %) wurde wie folgt kalkuliert:[(anti-CD3 + Inhibitor) - anti-CD3)]/[(Kontrolle) - (anti-CD3)] x 100.

Für die Bereitstellung einer gemischten Leukozytenkultur wurden Milzzellen aus Perforin-defizienten oder gld C57BL/6 Mäusen (H-2^{b}) mit Gamma-bestrahlten (36 Gy) Milzzellen aus Balb/c-Mäusen (H-2^{d}) für die Dauer von 5 Tagen kultiviert. Vor ihrem Einsatz wurden nicht-lebensfähige Zellen aus den Proben durch eine Gradienten-Zentrifugation auf Ficoll-Paque (von Pharmacia Biotech) entfernt. Die Markierung wurde nach vorhergehend beschriebenen Methoden (Kataoka et al. ) durchgeführt. Kurz gesagt, wurden Zielzellen (A20-Zellen) mit Natrium[⁵¹Cr]-Chromat (von Dupont, Boston, MA) für die Dauer von 1 Stunde markiert, dann dreimal mit RPMI 1640 gewaschen. MLC-Zellen wurden mit den Zielzellen (10⁴ Zellen pro "Well") in U-förmigen Mikrotiterplatten in Gegenwart oder Abwesenheit von Fas:Fc und Fas:COMP bei 40 µg/ml in einem Endvolumen von 200 µl gemischt und die Platten zentrifugiert (200xg, 3 min). Nach der Inkubation für 4 Stunden wurden die Überstände entfernt und deren Radioaktivität gemessen. Die spezifische [⁵¹Cr]-Freisetzung (in %) wurde mit Hilfe der folgenden formel bestimmt: [(experimentelle Freisetzung - spontaner Freisetzung)/(maximale Freisetzung - spontaner Freisetzung)] x 100.

### (K) FACS-Markierung

Für die FACS-Markierung wurde der CD40L⁺-Jurkat-Klon D 1.1 (5x10⁵ Zellen) mit 2µg von CD40:COMP in FACS-Puffer (PBS, 10% fötales Kälberserum und 0,02% NaN₃) inkubiert. Fas:COMP wurden als negative Kontrolle eingesetzt. Rezeptor:COMP wurde mit 1 µg von 9E10 anti-myc-Antikörper und nachfolgender Behandlung mit FITC markiertem Ziegen-anti-Maus-Antikörper (1:100) detektiert. Inkubationen wurde für die Dauer von 20 Min bei 4°C in 50 µl FACS-Puffer durchgeführt.

### (L) B-Zell-Proliferationstest

Für den B-Zell-Proliferationsassay wurden CD19⁺-Zellen aus humanen peripheren Blutlymphozyten (PBL) mit magnetischen "Beads" (Kügelchen) gereinigt und die übrigen CD19⁻⁻-Zellen bestrahlt (3000 rad). 10⁵ gereinigte CD19⁺-Zellen wurden mit 10⁵ CD19⁻ autologen bestrahlten PBL in 120 µl eines Mediums, das sCD40L bei einer Konzentration von 100 ng/ml (1,8 nM) M2-Antikörper bei 10 µg/ml plus oder minus Protein A bei einer Konzentration von 1 µg/ml und die angegebenen Konzentrationen von CD40:Fc oder CD40:COMP enthielt. Die Zellen wurden dann für 72 Stunden in 96 Well-Platten kultiviert, für 6 Stunden mit [³H]Thymidin (1 µCi/Well) gepulst und geerntet. Der [³H]Thymidin-Einbau wurde durch Flüssig-Szintillationszählung überprüft.

### 7.2 Ergebnisse des 7. Ausführungsbeispiels.

Fusionsproteine, die aus extrazellulären Domänen eines Rezeptors, der an den Fc-Teil von IgG (Rezeptor:Fc) fusioniert ist, bestehen, sind als inhibitorische Agenzien zur Erforschung von Rezeptorliganden-Wechselwirkungen aus dem Stand der Technik bekannt. Im 7. Ausführungsbeispiel wurde die Größe von gereinigtem Fas:Fc-Fusionsprotein durch Ausschlußchromatographie untersucht und herausgefunden, daß ein einzelner Peak mit der erwarteten Retentionszeit eluiert wurde. Fas:Fc enthaltene Fraktionen waren in der Lage, A20-Zellen, die einer letalen Dosis von löslichem FasL (sFasL) ausgesetzt waren, vor dem Zelltod zu schützen, obwohl das Maß an Protektion (bis 50%) außergewöhnlich niedrig war, wenn man berücksichtigt, daß das geschätzte Verhältnis von Fas:Fc zu FasL im Versuch ungefähr 1000 betrug.

Eine schwache protektive Aktivität wurde in einigen der frühen Fraktionen des Eluats beobachtet, die wahrscheinlich auf geringfügige nicht-detektierbare Mengen von hochmolekularen Komplexen von Fas:Fc enthielten. Erfindungsgemäß wurde erkannt, daß derartige höhere Aggregate des Fusionsproteins als potente Inhibitoren von FasL initiiertem Zelltod fungieren können. Daher wurde zunächst diese hochmolekulare Fraktion von aggregiertem Fas:Fc erhöht, indem das Immunogloguline kreuzvernetzende Agens Protein A hinzugegeben wurde. Sobald die Analyse unter Bedingungen durchgeführt wurde, bei denen nur ungefähr 10% des injizierten Fas:Fc zu den früh eluierenden Fraktionen verschoben wurde, war offensichtlich, daß hochmolekularer Fas:Fc-Komplex ein wirksamer Antagonist der FasL induzierten Zytotoxizität ist. Im Vergleich hierzu ergab sich, daß die übrigen Fas:Fc-Fraktionen immer noch als Dimer eluierten und nur teilweise Protektion für die Zellen trotz 10fach höherer Konzentration boten. Erfindungsgemäß zeigen diese Resultate des 7. Ausführungsbeispiels, daß die Bildung von höheren Fas:Fc-Aggregaten die spezifische protektive Aktivität wesentlich vergrößert.

Auf der Basis dieser Erkenntnisse wurde daher erfindungsgemäß Komplexe aus Fusionsproteinen konstruiert, die auf Grund einer Vergrößerung des Oligomerisierungsgrads von Fas eine bessere Avidität gegenüber FasL zeigen und die inhibitorischen Eigenschaften verbessern. Erfindungsgemäße Fusionsproteine sind bspw. solche, bei welchen die extrazellulären Domänen von Rezeptoren der TNF-Familie, bspw. Fas, TRAIL-R1, TRAIL-R2, TRAIL-R3, TNF-R1 oder CD40, über einen 14 Aminosäuren langen Linker an die oligomerisierenden Domänen von entweder dem sog. Cartilage Oligomeric Matrix Protein ((COMP); Fusionsprotein bezeichnet als: Rezeptor:COMP) oder dem sog. Cartilage Matrix Protein ((CMP); Fusionsprotein: Rezeptor:CMP) fusioniert sind. Diese Matrix-Proteine bzw. deren Domänen besitzen die native Eigenschaft, Pentamere bzw. Trimere coiled-coil-Strukturen zu bilden. Die vorgenannten Fusionsproteine (sowie auch Rezeptor:Fc-Fusionsproteine als Kontrollen) wurden in Säugetierzellen exprimiert und durch Affinitäts- chromatographie auf Metallchelatsäulen oder auf Protein A gereinigt. Die Rezeptoren Fas und TRAIL-R2 wurden im 7. Ausführungsbeispiel auch an die C-terminale Dimerisierungsdomäne des Proteins Osteoprotegerin aus der TNF Familie gebunden (Rezeptor:δN-OPG).

Die Rezeptoren, die an COMP oder CMP fusioniert waren, oligomerisierten, wie durch die langsame Wanderung im Polyacrylamidgel unter nicht-reduzierenden Bedingungen gezeigt werden konnte. Fas:COMP und TRAIL-R2:CMP eluierten als wohl definierte "Peaks" mit apparenten Molekulargewichten von ungefähr 400 bzw. 170 kDa bei Anwendung der Methode der Gel-Permeationschromatographie. Die Molekulargewichte stimmen mit pentameren bzw. trimeren Strukturen der Fusionsproteine überein. Aus den Experimenten des 7. Auasführungsbeispiels kann daher geschlossen werden, daß die Aggregationseigenschaften von coiled-coil Oligomerisierungsdomänen der vorgenannten Matrixproteine nicht durch die Fusion an Proteine (oder Proteindomänen) der TNF-Rezeptorfamilie beeinträchtigt werden.

Das Fusionsprotein Fas:COMP zeigte eine niedrigere K_{D} als Fas:Fc, wenn es den Fusionsproteinen gestattet wurde, um die sFasL-Bindung gegenüber aufgetragenem Fas:Fc in Kompetition zu treten. In Übereinstimmung mit diesem Resultat wurde eine Dissoziationskonstante von 0,77 nM für die FasL-Fas:COMP-Wechselwirkung gemessen, die ungefähr 8 bis 9 mal niedriger ist als Vergleichswerte für Fas:Fc. Bei der großen Mehrzahl der getesteten Zellinien zeigte sich, daß die inhibitorische Aktivität von Fas:COMP ungefähr 10- bis 20-fach höher ist als jene für das dimere Fusionsprotein Fas:Fc, während die Ergebnisse für Fas:Fc-Aggregate, hervorgerufen durch kreuzvernetzendes Protein A (Fas:Fc/PA), Werte zwischen jenen für Fas:COMP und Fas:Fc zeigten. Die protektive Aktivität von dimeren Fas:δN-OPG-Fusionsprotein war vergleichbar mit jener des dimeren Fas:Fc-Komplexes. Trimeres Fas:CMP inhibierte die sFasL vermittelte Lyse ungefähr so wirksam wie Fas:Fc/PA - im Ergebnis also 5x weniger wirksam als Fas:COMP. Die inhibitorische Aktivität von Fas:COMP war gut oder besser als jene von FasL blockierenden monoklonalen Antikörpern Nok-1, 4H9 und 4A5. Die überlegene inhibitorische Aktivität von Fas:COMP, verglichen mit Fas:Fc-Komplex, war auch ersichtlich aus Versuchen mit primären Maus-Hepatocyten oder aus einem Modellsystem für den aktivierungsinduzierten Zelltod mit anti-CD3 aktivierten Jurkat-Zellen. In all diesen Experimenten ergab sich für Fas:Fc/PA ein mittlerer Protektionswert. Weiterhin wurde untersucht, ob Fas:COMP die Wirkung von FasL, exprimiert auf CTLs, inhibieren kann. Der Tod von A20-Zellen in einem 4stündigen Testsystem hängt einzig von Perforin und den FasL-abhängigen Signaltransduktionswegen ab, weil CTLs, die sowohl für Perforin als auch für FasL defizient sind, auf diese Zellen keine Wirkung haben. In einem entsprechenden Versuch wurden A20-Zellen erwartungsgemäß sowohl durch Perforin-defiziente CTLs als auch durch FasL-defiziente CTLs getötet. Fas:Fc und Fas:COMP riefen spezifisch ein gewisses Maß an Protektion für die Zellen, die Perforin-defizienten CTLs ausgesetzt waren, hervor.

Bei einem Vergleich der Affinitäten von sCD40L an CD40:Fc, CD40:FC/PA oder erfindungsgemäßes CD40:COMP durch Kompetitions-ELISA wurde eine beträchtliche Steigerung der Affinität (30-fach) für den pentamerisierten Rezeptor beobachtet, während sich wiederum ein mittlerer Effekt für CD40:Fc/PA (8-fach) einstellte. Hinsichtlich der Frage, ob CD40:COMP auch membrangebundenen CD40L erkennen kann, wurde die Jurkat-derivatisierte Zellinie D1.1, die konstitutiv das Oberflächenprotein CD40L exprimiert, für die Durchführung einer FACS-Markierungsanalyse mit Hilfe der CD40-Fusionsproteine als Proben durchgeführt. Eine signifikante Markierung wurde für erfindungsgemäßes CD40:COMP beobachtet, wodurch indiziert wird, daß CD40-COMP in der Tat zur Bindung an natives nicht-prozessiertes CD40L in der Lage ist. Um die spezifische Aktivität von CD40-Fusionsprotein in einem biologischen System zu untersuchen, wurde versucht, die CD40L abhängige Proliferation von humanen B-Zellen, die mit anti-B-Zell-Rezeptor costimuliert worden sind, zu inhibieren. Weder CD40:Fc noch CD40:Fc/PA konnten die Proliferation signifikant beeinträchtigen, sogar für den Fall, daß sie in hohen Dosen verabreicht wurden. Im Gegensatz hierzu war erfindungsgemäßes CD40:COMP in der Lage, die Proliferation bereits bei relativ geringen Dosen zu blockieren.

Zusammenfassend kann aus den Untersuchungen des vorliegenden Ausführungsbeispiels festgestellt werden, daß die Inhibition von FasL-induzierter Apoptose durch kompetive Inhibitoren abhängig von dem Oligomerisierungsgrad zunahm und die relativen Aktivitäten der verschiedenen Inhibitoren (ausgedrückt als Verhältnis ihrer jeweiligen IC₅₀-Werte) relativ konstant für die verschiedenen Zellinien blieb. Die erhöhte inhibitorische Wirksamkeit von erfindungsgemäßem Fas:COMP im Vergleich zu Fas.Fc ist wahrscheinlich das Ergebnis seiner höheren Avidität. Ähnliche Ergebnisse konnten auch für die Aktivität von erfindungsgemäßem CD40-Fusionsprotein bestimmt werden. Erfindungsgemäßes CD40:COMP ist CD40:Fc bei der Inhibition von CD40L-induzierter Proliferation von primären D-Zellen in vitro deutlich überlegen. Damit zeigen die Resultate des Ausführungsbeispiels 7, daß die Dissoziationskonstanten im niedrigen nanomolaren Bereich für solche Rezeptoren, wie z. B. Fas und CD40, erhalten werden können, die sich nativ durch mittlere Affinität für ihre Liganden auszeichnen, wenn sie - wie erfindungsgemäß vorgesehen, Bestandteil von Fusionsproteinen sind, die sich durch einen höhereren Oligomerisierungsgrad auszeichnen.

## Patentansprüche

1. Fusionsprotein, **dadurch gekennzeichnet, daß** das rekombinante Fusionsprotein eine Komponente A und eine Komponente B enthält, wobei
die Komponente A ein extrazellulärer Abschnitt eines TNF-Cytokins ist, und
die Komponente B einen multimerisierenden und oligomerisierenden Abschnitt ausgewählt aus der Gruppe, bestehend aus Clq, MBP, SP-A, SP-D, BC, CL-43 und ACRP30 das der Fusionsprotein ohne Wirkung von Drittmolekülen multimerisiert und oligomerisiert, enthält.

2. Fusionsprotein nach Anspruch 1, **dadurch gekennzeichnet, daß** die Komponente B einen multi- und oligomerisierenden Abschnitt eines ACRP30 Proteins enthält.

3. Fusionsprotein nach einem der Ansprüche 1 bis 2, **dadurch gekennzeichnet, daß** die Komponente B eine Aminosäuresequenz, wie gemäß Fig. 6A für die Sequenz von Aminosäure 18 bis 111 von mACRP30 angegeben oder wie gemäß Fig. 6B für die Sequenz von Aminosäure 18 bis 108 von hACRP30 angegeben.

4. Fusionsprotein nach einem der Ansprüche 1 bis 3, **dadurch gekennzeichnet, daß** die Komponente A ein Abschnitt eines TNF-Cytokins, ausgewählt aus der Gruppe, bestehend aus CD40L, FasL, TRAIL, TNFα, CD30L, OX40L, RANKL, TWEAK, Lta, Ltab2, LIGHT, CD27L, 41-BB, GITRL, APRIL, EDA, VEGI und BAFF, ist.

5. Fusionsprotein nach einem der Ansprüche 1 bis 4, **dadurch gekennzeichnet, daß** die Komponente A ein Abschnitt eines TNF-Cytokins, ausgewählt aus der Gruppe, bestehend aus hFasL (AA 139-261), hTRAIL (AA 95-281), hCD40L (AA 116-261) und/oder hTNFα (AA 77-235) ist.

6. Fusionsprotein nach einem der Ansprüche 1 bis 5, **dadurch gekennzeichnet, daß** die rekombinanten Fusionsproteine zwischen Komponente A und Komponente B eine Linkersequenz aufweisen.

7. Bi- oder Oligomer von rekombinanten Fusionsproteinen nach einem der Ansprüche 1 bis 6.

8. DNA-Sequenz, **dadurch gekennzeichnet, daß** die DNA Sequenz für ein Fusionsprotein nach einem der Ansprüche 1-6 codiert.

9. Expressionsvektor, **dadurch gekennzeichnet, daß** der Expressionsvektor eine DNA-Sequenz nach Anspruch 8 enthält.

10. Wirtszelle, **dadurch gekennzeichnet, daß** die Wirtszelle mit einem Expressionvektor nach Anspruch 9 transfiziert ist.

11. Verwendung von Bi- oder Oligomeren nach Anspruch 7 zur Herstellung eines Arzneimittels.

12. Verwendung von Bi- oder Oligomeren nach Anspruch 7 zur Herstellung eines Arzneimittels zur Behandlung von hyperinflammatorischen Störungen, Autoimmunerkrankungen, Erkrankungen, beruhend auf hyper- oder hypoapoptotischen Störungen, von Infektionserkrankungen, insbesondere viralen Infektionserkrankungen, Tumorerkrankungen, insbesondere Tumoren des Lymphsystems und/oder endokrinologischen Störungen.

13. Verwendung von Bi- oder Oligomeren nach Anspruch 7 zur Herstellung eines Arzneimittels zur parenteralen oder oralen Verabreichung.

14. Verwendung von Bi- oder Oligomeren nach Anspruch 7 zur In-vitro-Diagnose.

15. Arzneimittel **dadurch gekennzeichnet, daß** der Arzneimittel einem Bi- oder Oligomer von rekombinanten Fusionsproteinen nach Anspruch 7, enthält.
